# EUROPEAN PATENT APPLICATION

(11) **EP 3 108 924 A1**
(43) Date of publication of application: **28.12.2016**
(21) Application number: 15767856.6
(22) Date of filing: 25.03.2015
(51) Int. Cl.: A61M 37/00, B65D 75/32, B65D 77/20, B65D 81/02, B65D 85/00

(54) **PACKAGING BODY FOR SHEET WITH CONICAL PROJECTIONS AND METHOD FOR MANUFACTURING SAME**

(30) Priority: 26.03.2014 JP 2014063849; 26.03.2014 JP 2014063875
(71) Applicant: Nissha Printing Co., Ltd., Kyoto-shi, Kyoto 604-8551 (JP)
(72) Inventor: YAMADA, Shinya, Kyoto-shi Kyoto 604-8551 (JP); UENO, Takako, Kyoto-shi Kyoto 604-8551 (JP); KAEDE, Chika, Kyoto-shi Kyoto 604-8551 (JP); NAGAI, Hiroyuki, Kyoto-shi Kyoto 604-8551 (JP); KATOU, Yuki, Kyoto-shi Kyoto 604-8551 (JP); NISHIMURA, Yuka, Kyoto-shi Kyoto 604-8551 (JP)
(74) Representative: Kastel, Stefan
(86) International application number: PCT/JP2015/059049
(87) International publication number: WO 2015/147030

(57) **Abstract**

A conical-projections-sheet packaging body (1) includes a conical-projections sheet (23), a conical-projections forming sheet (7), and a sealing sheet (9). The conical-projections sheet (23) includes a substrate (23a) and a plurality of conical projections (23b) formed on a surface (23c) of the substrate (23a). The conical-projections forming sheet (7) has a plurality of conical-recess parts (33a), which is used to form the plurality of conical projections (23b) and wherein the plurality of conical projections (23b) is disposed. The conical-projections forming sheet (7) protects the conical projections (23b) in a state wherein the plurality of conical projections (23b) is tightly adhered to the surface (23c) of the substrate (23a). The sealing sheet (9), by being fixed to the conical-projections forming sheet (7), seals the conical-projections sheet (23) between the conical-projections forming sheet (7) and the sealing sheet (9).

## Description

### TECHNICAL FIELD

The present invention relates to a conical-projections-sheet packaging body including a plurality of conical projections, and to a method of manufacturing the same.

### BACKGROUND ART

A resin sheet having a plurality of minute recessed parts is utilized as, for example, a conical-projections forming mold for manufacturing a plurality of conical projections on a conical-projections sheet (e.g., refer to Patent Citation 1). Furthermore, one example of a conical-projections sheet is a transdermal patch. A transdermal patch is used as one means of noninvasively administering a drug or the like via the body surface of an organism, such as the skin or a mucous membrane. In such a case, the drug is adhered to the conical projections.

### CITATION LIST

### PATENT CITATIONS

Patent Citation 1
PCT International Publication No. WO2011/002034

### SUMMARY OF INVENTION

### TECHNICAL PROBLEM

Each conical projection of the plurality of conical projections of the conical-projections sheet has a minute shape that is relatively easily damaged. Furthermore, after a completed conical-projections sheet is packaged and ready to be shipped and transported, it is necessary to take measures to insure that the conical projections do not get damaged. However, an effective measure to protect the conical projections inside the packaging has yet to be proposed.

When the conical-projections sheet is to be used, the user, for example, peels off a cover of a blister pack, then removes the transdermal patch from the blister pack, and lastly affixes such to his or her own skin. In such a case, because the transdermal patch is removed directly from the blister pack, there is a possibility that the plurality of conical projections will get damaged if the removal procedure is difficult.

An object of the present invention is to protect a plurality of conical projections of a conical-projections sheet.

An object of the present invention is to make it such that, when a package is removed from the conical-projections sheet and used, the plurality of conical projections tends not to get damaged.

### TECHNICAL SOLUTION

Aspects of the invention are explained below as the technical solution. These aspects can be arbitrarily combined as needed.

A conical-projections-sheet packaging body according to one aspect of the present invention includes a conical-projections sheet, a conical-projections forming sheet, and a sealing sheet. The conical-projections sheet includes a substrate and a plurality of conical projections formed on a surface of the substrate. The conical-projections forming sheet has a plurality of conical-recess parts, which is used to form the plurality of conical projections and where the plurality of conical projections is disposed. The conical-projections forming sheet protects the plurality of conical projections in a state wherein the plurality of conical projections is tightly adhered to the surface of the substrate. The sealing sheet, by being fixed to the conical-projections forming sheet, seals the conical-projections sheet between the conical-projections forming sheet and the sealing sheet in the conical-projections-sheet packaging body.

In this case, the plurality of conical projections of the conical-projections sheet is formed by the plurality of conical-recess parts of the conical-projections forming sheet; subsequently, too, the state wherein the plurality of conical projections is protected by the plurality of conical-recess parts is maintained. That is, the conical projections of the conical-projections sheet are reliably protected.

In addition, because the conical-projections sheet is sealed between the conical-projections forming sheet and the sealing sheet, the quality of the conical-projections sheet is maintained for a long time. As discussed above, because the conical-projections forming sheet, together with the sealing sheet, is used as the member that constitutes the packaging body, the part count of the conical-projections-sheet packaging body can be reduced while the plurality of conical projections is reliably protected.

The conical-projections forming sheet may have a recessed-housing part wherein the plurality of conical-recess parts is formed; and the conical-projections sheet may be disposed inside the recessed-housing part.

The conical-projections-sheet packaging body may further include: a support body that, by being fixed to a second surface of the substrate of the conical-projections sheet and further being adhered to the conical-projections forming sheet, maintains a state wherein the plurality of conical projections of the conical-projections sheet is disposed inside the plurality of conical-recess parts of the conical-projections forming sheet.

In this case, by virtue of the support body being adhered to the conical-projections forming sheet, the state is maintained wherein the plurality of conical projections of the conical-projections sheet is disposed inside the plurality of conical-recess parts of the conical-projections forming sheet. Furthermore, when the support body is peeled from the conical-projections forming sheet, the conical-projections sheet can be separated from the conical-projections forming sheet together with the support body.

In the conical-projections-sheet packaging body, a process of reducing peel strength may be performed on a portion of the conical-projections forming sheet at which the support body is adhered.

In this case, the portion of the conical-projections forming sheet at which the support body is adhered is subject to the process of reducing the peel strength, and therefore the support body, together with the conical-projections sheet, easily peels from the conical-projections forming sheet without reducing the strength with which the support body adheres to the human body or other members. Accordingly, when the conical-projections sheet is removed and used, the plurality of conical projections tends not to get damaged.

The process of reducing the peel strength may be performed by applying a die-releasing agent or using a material that exhibits die-releasing effect, or both.

The peel strength between the support body and the sealing sheet may be 2.0-6.5 (N/10 mm).

The peel strength between the support body and the conical-projections forming sheet may be 0.5-4.0 (N/10 mm).

A method of manufacturing a conical-projections-sheet packaging body according to another aspect of the present invention includes the following steps:
- a step of forming a plurality of conical-recess parts on a conical-projections forming sheet;
- a step of forming, by supplying a conical-projections material to the conical-projections forming sheet, a conical-projections sheet including a substrate and a plurality of conical projections formed on a surface of the substrate and disposed inside the plurality of conical-recess parts; and
- a step of sealing the conical-projections sheet between the conical-projections forming sheet and the sealing sheet by fixing the sealing sheet to the conical-projections forming sheet.

In this manufacturing method, the conical-projections sheet is formed in the conical-projections forming sheet, and that state is maintained. Specifically, the plurality of conical projections of the conical-projections sheet is formed by the plurality of conical-recess parts of the conical-projections forming sheet and is subsequently protected by the plurality of conical-recess parts. That is, the plurality of conical projections of the conical-projections sheet is reliably protected.

In addition, because the conical-projections sheet is sealed between the conical-projections forming sheet and the sealing sheet, the quality of the conical-projections sheet is maintained for a long time. As discussed above, because the conical-projections forming sheet, together with the sealing sheet, is used as the member that constitutes the packaging body, the part count of the conical-projections-sheet packaging body can be reduced while the plurality of conical projections is reliably protected.

The method of manufacturing may further include: a step of forming, prior to forming the plurality of conical-recess parts, a recessed-housing part at a location at which the plurality of conical-recess parts is to be formed on the conical-projections forming sheet. In that case, in the step of forming the conical-projections sheet, the conical-projections sheet is formed inside the recessed-housing part.

The method of manufacturing may further include: a step that, by fixing a support body to a second surface of the substrate of the conical-projections sheet and further adhering the support body the conical-projections forming sheet, maintains a state wherein the plurality of conical projections of the conical-projections sheet is disposed inside the plurality of conical-recess parts of the conical-projections forming sheet.

In this case, by virtue of the support body being adhered to the conical-projections forming sheet, the state is maintained wherein the plurality of conical projections of the conical-projections sheet is disposed inside the plurality of conical-recess parts of the conical-projections forming sheet. Furthermore, when the support body is peeled from the conical-projections forming sheet, the conical-projections sheet, together with the support body, is separated from the conical-projections forming sheet.

The method of manufacturing the conical-projections-sheet packaging body may further include the following step:
- a step of performing, prior to the step of forming the conical-projections sheet, a process of reducing peel strength at a portion of the conical-projections forming sheet at which the support body is to be adhered.

In this case, because the process for reducing the peel strength is performed at the portion of the conical-projections forming sheet at which the support body is adhered, the support body, together with the conical-projections sheet, is easy to peel from the conical-projections forming sheet without reducing the strength with which the support body adheres to the human body or other members. Accordingly, when the conical-projections sheet is removed from the conical-projections forming sheet and used, the plurality of conical projections tends not to get damaged.

Furthermore, unless particularly stated, the order of the steps is not limited. In addition, the steps may be performed simultaneously or partially overlapping.

The process of reducing the peel strength may be performed by applying a die-releasing agent or using a material that exhibits a die-releasing effect, or both.

### ADVANTAGEOUS EFFECTS

In a conical-projections-sheet packaging body and a method of manufacturing the same according to the present invention, a plurality of conical projections of a conical-projections sheet can be protected.

### BRIEF DESCRIPTION OF DRAWINGS

FIG. 1 is a partial plan view of a conical-projections-sheet packaging body according to a first embodiment of the present invention.
FIG. 2 is a cross-sectional view taken along line II-II in FIG. 1.
FIG. 3 is a cross-sectional view that shows a state wherein a sealing sheet has been peeled from a conical-projections forming sheet.
FIG. 4 is a cross-sectional view that shows a state wherein a support-body-attached, conical-projections sheet has been peeled from the sealing sheet.
FIG. 5 is a cross-sectional view of a blank of the conical-projections forming sheet.
FIG. 6 is a schematic drawing that illustrates cleaning, drying, and preheating operations.
FIG. 7 is a schematic drawing that illustrates an operation of forming conical-recess parts.
FIG. 8A is a schematic drawing of a conical-projections forming sheet before the conical-recess parts have been formed.
FIG. 8B is a partial cross-sectional view of the conical-projections forming sheet before the conical-recess parts have been formed.
FIG. 9 is a cross-sectional view for explaining a process of forming the conical-recess parts on the conical-projections forming sheet.
FIG. 10A is a schematic drawing of the conical-projections forming sheet after the conical-recess parts have been formed.
FIG. 10B is a partial cross-sectional view of the conical-projections forming sheet after the conical-recess parts have been formed.
FIG. 11 is a schematic drawing for explaining a process of dripping conical-projections materials onto the conical-projections forming sheets.
FIG. 12 is a partial cross-sectional view of the conical-projections forming sheet filled with the conical-projections material.
FIG. 13 is a schematic drawing for explaining a process of drying the conical-projections materials.
FIG. 14 is a schematic drawing for explaining a process of temporarily fixing the sealing sheets to the conical-projections forming sheets.
FIG. 15 is a cross-sectional view of the sealing sheet and the conical-projections forming sheet.
FIG. 16 is a cross-sectional view of a state wherein the sealing sheet has been temporarily fixed to the conical-projections forming sheet.
FIG. 17 is a schematic drawing for explaining a process of fixing the sealing sheets to the conical-projections forming sheets and a process of cutting up the completed conical-projections-sheets packaging bodies.
FIG. 18 is a cross-sectional view of the microneedle-sheet packaging body according to a second embodiment.
FIG. 19 is a cross-sectional view that shows a state wherein the sealing sheet has been peeled from the conical-projections forming sheet.
FIG. 20 is a cross-sectional view that shows a state wherein the support-body-attached, conical-projections sheet has been peeled from the sealing sheet.
FIG. 21 is a cross-sectional view of a blank of the conical-projections forming sheet.
FIG. 22A is a schematic drawing that shows a process of coating a stepped surface with a die-releasing agent.
FIG. 22B is a schematic drawing that shows the process of coating the stepped surface with the die-releasing agent.
FIG. 22C is a schematic drawing that shows the process of coating the stepped surface with the die-releasing agent.
FIG. 23 is a partial cross-sectional view that shows the conical-projections forming sheet before the conical-recess parts have been formed.
FIG. 24 is a cross-sectional view for explaining a process of forming the conical-recess parts on the conical-projections forming sheet.
FIG. 25 is a partial cross-sectional view of the conical-projections forming sheet after the conical-recess parts have been formed.
FIG. 26 is a partial cross-sectional view of the conical-projections forming sheet filled with the conical-projections material.
FIG. 27 is a cross-sectional view of the sealing sheet and the conical-projections forming sheet.
FIG. 28 is a cross-sectional view of a state wherein the sealing sheet has been temporarily fixed to the conical-projections forming sheet.
FIG. 29 is a cross-sectional view of the conical-projections-sheet packaging body according to a third embodiment.
FIG. 30 is a cross-sectional view of the conical-projections-sheet packaging body according to a fourth embodiment.
FIG. 31A is a schematic drawing that shows a process of embossing the stepped surface.
FIG. 31B is a schematic drawing that shows the process of embossing the stepped surface.
FIG. 31C is a schematic drawing that shows the process of embossing the stepped surface.
FIG. 32 is a cross-sectional view of the conical-projections-sheet packaging body according to a fifth embodiment.
FIG. 33 is a schematic drawing for explaining, according to the fifth embodiment, a process of temporarily fixing the sealing sheet to the conical-projections forming sheet.
FIG. 34 is a plan view of the microneedle sheet packaging body according to a sixth embodiment of the present invention.
FIG. 35 is a cross-sectional view taken along line XXXV-XXXV in FIG. 34.
FIG. 36 is an oblique view of a blank of a microneedle-forming sheet.
FIG. 37 is a cross-sectional view of a blank of the microneedle-forming sheet.
FIG. 38 is a schematic drawing that illustrates a preheating and forming process.
FIG. 39 is a cross-sectional view of a press die.
FIG. 40 is a schematic cross-sectional view that shows a state before the formation of the blank.
FIG. 41 is a schematic cross-sectional view that shows a state after the formation of the blank.
FIG. 42 is a schematic drawing for explaining a process wherein microneedle materials are dripped onto the microneedle-forming sheets.
FIG. 43 is a schematic cross-sectional view of the microneedle-forming sheet filled with the microneedle material.
FIG. 44 is a schematic drawing for explaining a process of drying the microneedle materials.
FIG. 45 is a schematic cross-sectional view of the microneedle-forming sheet filled with the microneedle material before the drying process.
FIG. 46 is a schematic cross-sectional view of the microneedle-forming sheet filled with the microneedle material after the drying process.
FIG. 47 is a cross-sectional view of a conical-projections-sheet, forming-and-protecting-structure preform.
FIG. 48 is a schematic cross-sectional view for explaining a process of manufacturing the packaging body from the conical-projections-sheet, forming-and-protecting-structure preform.
FIG. 49 is a schematic cross-sectional view for explaining a process of manufacturing the packaging body from the conical-projections-sheet, forming-and-protecting-structure preform.
FIG. 50 is a plan view of the conical-projections-sheet, forming-and-protecting-structure preform.
FIG. 51 is a schematic cross-sectional view for explaining a process of manufacturing the packaging body from the conical-projections-sheet, forming-and-protecting-structure preform.
FIG. 52 is a plan view of the conical-projections-sheet, forming-and-protecting-structure preform.
FIG. 53 is a schematic cross-sectional view for explaining a process of manufacturing the packaging bodies from the conical-projections-sheet, forming-and-protecting-structure preform.
FIG. 54 is a schematic cross-sectional view for explaining the process of manufacturing the packaging bodies from the conical-projections-sheet, forming-and-protecting-structure preform.
FIG. 55 is a plan view of the conical-projections-sheet, forming-and-protecting-structure preform.

### DESCRIPTION OF EMBODIMENTS

### 1. First Embodiment

### (1) Conical-Projections-Sheet Packaging Body

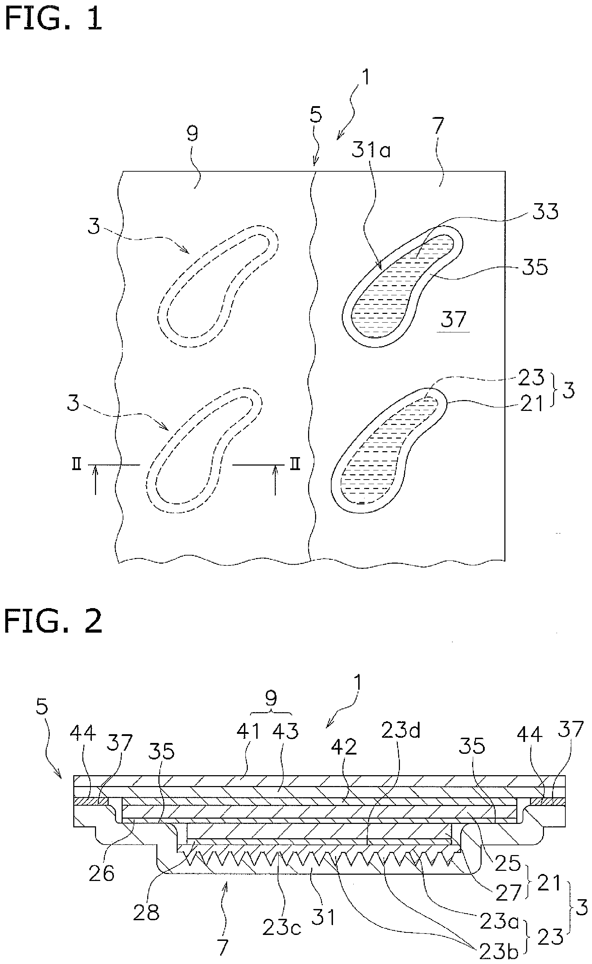

A conical-projections-sheet packaging body 1 according to a first embodiment will be explained, with reference to FIG. 1 and FIG. 2. FIG. 1 is a partial plan view of the conical-projections-sheet packaging body according to the first embodiment of the present invention. FIG. 2 is a cross-sectional view taken along line II-II in FIG. 1.

The conical-projections-sheet packaging body 1 includes a plurality of support-body-attached, conical-projections sheets 3 and a packaging material 5, which packages the support-body-attached, conical-projections sheets 3. Furthermore, in the embodiment below, a plurality of the support-body-attached, conical-projections sheets 3 is housed inside the conical-projections-sheet packaging body 1, but there may be only a single support-body-attached, conical-projections sheet 3.

### (1-1) Support-Body-Attached, Conical-Projections Sheet

The support-body-attached, conical-projections sheets 3 are members that are packaged by the packaging material 5 and are used by being removed from the packaging material 5 as needed. Each support-body-attached, conical-projections sheet 3 is a sheet-shaped article that principally includes a support body 21 and a conical-projections sheet 23.

The conical-projections sheet 23 is a sheet-shaped article whereon a plurality of conical projections are formed on one surface, which is discussed below. The thickness of the conical-projections sheet 23 is approximately several hundred micrometers. The overall planar shape of the conical-projections sheet 23 is smoothly curved and has a magatama (comma) shape having a portion with a large width and a portion with a small width. The shape of the conical-projections sheet 23 may be a circle, an ellipse, a triangle, a quadrangle, a square, or some other shape. If the conical-projections sheet 23 is, for example, a quadrangle, then one side is approximately several to several tens of millimeters.

The structure of the conical-projections sheet 23 will now be explained further. The conical-projections sheet 23 includes a sheet-shaped substrate 23a and a plurality of conical projections 23b. The plurality of conical projections 23b is formed on a surface 23c (in the present embodiment, a lower surface) of the substrate 23a. Each conical projection 23b has, for example, a conical shape or a pyramidal shape with a height of 10-1000 µm, wherein the ratio of the cross-sectional diameter, at the base, to the height is 1:0.2-1:5 and the aspect ratio (height/cross-sectional diameter) is high.

The conical-projections sheet 23 is a transdermal patch that is used, for example, to administer a drug or the like by affixing the patch such that it contacts the skin of a person. Specifically, the administration of the drug or the like is promoted by the substrate 23a being stuck onto the skin and the conical projections 23b piercing the skin.

The conical-projections sheet 23 consists of, as the principal material, for example, a water-soluble drug, or, a water-soluble macromolecule, such as hyaluronate, a water-soluble collagen, dextran, chondroitin sulfate, or the like, to which a drug is added. Furthermore, the water-soluble macromolecule to which a pharmaceutical agent has been added is preferably an in-vivo-soluble, water-soluble macromolecule, for example, an in-vivo-soluble, water-soluble macromolecule such as sodium chondroitin sulfate, hyaluronate, or dextran.

Furthermore, the water-soluble macromolecule is a substance of at least 1 selected from the group consisting of sodium chondroitin sulfate, hyaluronate, a collagen (or a hydrolyzed collagen), gelatin, a glycogen, dextran, dextrin, dextran sulfate, cyclodextrin, chitosan, proteoglycan, pullulan, hydroxypropylcellulose, alginic acid, agarose, glycogen, carboxymethyl cellulose, hydroxyethyl cellulose, hydroxypropylcellulose, polyvinylpyrrolidone, polyvinyl alcohol, and a carboxyvinyl polymer. One of these water-soluble macromolecules may be used alone, or a plurality thereof may be used in combination.

The support body 21 is a sheet for supporting the conical-projections sheet 23 and is made of, for example, a resin. The support body 21 is fixed to a second surface 23d of the substrate 23a of the conical-projections sheet 23 and is further adhered to a conical-projections forming sheet 7.

The support body 21 principally includes a first sheet 25 and a second sheet 27. The first sheet 25 is disposed on the upper side, in the figure, of the second sheet 27 and has a surface area larger than that of the second sheet 27. The second sheet 27 is fixed to the lower surface of the first sheet 25 by a first adhesive layer 26. A first bonding layer 28 is provided on the lower surface of the second sheet 27. The support body 21 is fixed to the second surface 23d of the conical-projections sheet 23 by the first bonding layer 28.

Furthermore, the first adhesive layer 26 is provided on the lower surface of the first sheet 25 also at a portion further on the outer-perimeter side of the second sheet 27. Accordingly, the support body 21 (that is, the support-body-attached, conical-projections sheet 3) is fixed to the conical-projections forming sheet 7 by the first adhesive layer 26.

### (1-2) Packaging Material

The packaging material 5 is a member for packaging the support-body-attached, conical-projections sheets 3. The packaging material 5 includes the conical-projections forming sheet 7 and a sealing sheet 9; the support-body-attached, conical-projections sheets 3 are packaged by the two sheets being pasted together.

The conical-projections forming sheet 7 is a sheet-shaped member and has a plurality of housing parts 31 for housing the support-body-attached, conical-projections sheets 3. Each housing part 31 has a protruding shape that bulges toward the outer side of the packaging material 5, and a recessed-housing space 31 a is formed on the inner side of the packaging material 5. The planar shape of the recessed-housing space 31 a is the same as the planar shape of the support-body-attached, conical-projections sheet 3. The recessed-housing space 31 a has a bottom surface 33 and therearound a ring-shaped stepped surface 35 that is higher than the bottom surface 33. A flat surface 37 is provided around the recessed-housing space 31 a (that is, around the stepped surface 35). Thus, the recessed-housing space 31 a, which has a recessed shape, of the housing part 31 has a stepped shape that is formed by the bottom surface 33, the ring-shaped stepped surface 35, and the flat surface 37. As discussed above, the outer-perimeter part of the support body 21 is adhered to the ring-shaped stepped surface 35 via the first adhesive layer 26. Furthermore, the stepped surface 35 may be coated with a die-releasing agent to make it easy for the outer-perimeter part of the support body 21 to be peeled off.

Taking into consideration that the conical-projections forming sheet 7 is used as a member whereon a plurality of conical-recess parts 33a (discussed below) is to be formed, the conical-projections forming sheet 7 has a monolayer structure prepared with a polyolefin based resin such as polyethylene, polypropylene, or the like. However, the conical-projections forming sheet 7 may include a plurality of layers.

The plurality of conical-recess parts 33a, which is for forming and housing the conical projections 23b of the conical-projections sheet 23, is formed on the bottom surface 33 of the conical-projections forming sheet 7. Furthermore, the conical-recess part 33a is an indentation that is upwardly open but does not pass through the conical-projections forming sheet 7.

The support-body-attached, conical-projections sheet 3 is housed in the recessed-housing space 31 a of the housing part 31. Specifically, the conical-projections sheet 23 is formed in a state wherein it is tightly adhered to the bottom surface 33 and thereby the bottom surface 33 is in a state wherein it is tightly adhered to the surface 23c of the substrate 23a of the conical-projections sheet 23. In this state, the conical projections 23b are disposed inside the conical-recess parts 33a. Based on the above, the bottom surface 33 functions as a formation surface and a protective surface of the conical projections 23b.

In addition, as discussed above, by virtue of the support body 21 being adhered to the conical-projections forming sheet 7, the state is maintained wherein the plurality of conical projections 23b of the conical-projections sheet 23 is disposed in the plurality of conical-recess parts 33a of the conical-projections forming sheet 7. Furthermore, when the support-body-attached, conical-projections sheet 3 is removed from the conical-projections forming sheet 7, the outer-perimeter part of the support body 21 (the portion fixed by the first adhesive layer 26) can be easily peeled from the conical-projections forming sheet 7. Furthermore, the first adhesive layer 26 functions as an adhesive portion after the support-body-attached, conical-projections sheet 3 has been taken out.

The sealing sheet 9 is a sheet-shaped member that is stuck to the inner-side surface of the conical-projections forming sheet 7 in order to seal the support-body-attached, conical-projections sheet 3 inside the recessed-housing space 31a between the sealing sheet 9 and the conical-projections forming sheet 7. Specifically, the sealing sheet 9 includes a surface-resin layer 41 and a metal layer 43.

The surface-resin layer 41 is capable of being printed upon and further functions to protect the metal layer 43.

The metal layer 43 is provided on a lower surface of the surface-resin layer 41. The metal layer 43 includes aluminum or an alloy thereof and has a high degree of moisture proofness.

A second adhesive layer 42 and a second bonding layer 44 are provided on the surface of the metal layer 43 on the side opposite the surface-resin layer 41. The second adhesive layer 42 is for the purpose of adhering the support body 21 to the sealing sheet 9. The second bonding layer 44 is for the purpose of bonding the sealing sheet 9 to the conical-projections forming sheet 7; specifically, the second bonding layer 44 is bonded to the flat surface 37 of the conical-projections forming sheet 7.

Thus, the sealing sheet 9 is stuck to the inner-side surface of the conical-projections forming sheet 7 in a state wherein the sealing sheet 9 covers the conical-projections sheet 23, and thereby both seal the recessed-housing space 31 a.

In the conical-projections-sheet packaging body 1 discussed above, the conical projections 23b of each conical-projections sheet 23 are formed by the plurality of conical-recess parts 33a of the conical-projections forming sheet 7 (discussed below) and subsequently are also protected by the conical-projections forming sheet 7.

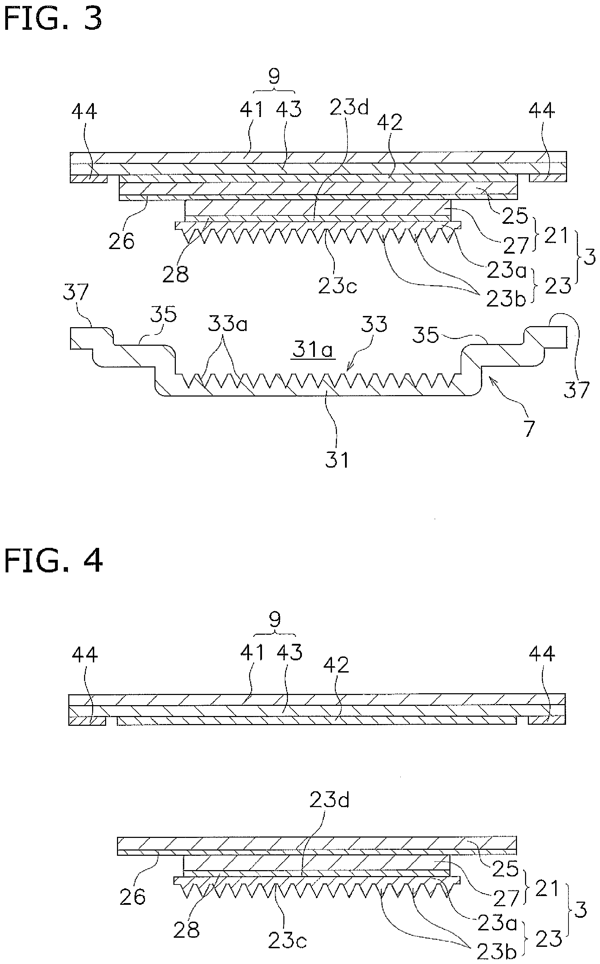

As shown in FIG. 3, when removing the support-body-attached, conical-projections sheet 3 from the conical-projections-sheet packaging body 1, a user peels the sealing sheet 9 from the conical-projections forming sheet 7. FIG. 3 is a cross-sectional view that shows a state wherein the sealing sheet has been peeled from the conical-projections forming sheet. At this time, as shown in FIG. 3, the support-body-attached, conical-projections sheet 3, together with the sealing sheet 9, is separated from the conical-projections forming sheet 7. The support-body-attached, conical-projections sheet 3 is removed from the recessed-housing parts 31a of the conical-projections forming sheet 7 by the aforementioned operation.

Next, as shown in FIG. 4, the user peels the support-body-attached, conical-projections sheet 3 from the sealing sheet 9. FIG. 4 is a cross-sectional view that shows a state wherein the support-body-attached, conical-projections sheet has been peeled from the sealing sheet. As a result, the user becomes able to use the support-body-attached, conical-projections sheet 3 in accordance with its purpose. In the case of a transdermal patch, the support-body-attached, conical-projections sheet 3 is stuck to the skin of a person. In this case, the support body 21 sticks to the skin via the first adhesive layer 26, and the conical projections 23b of the first adhesive layer 26 contact the skin.

### (2) Method of Manufacturing the Conical-Projections-Sheet Packaging Body

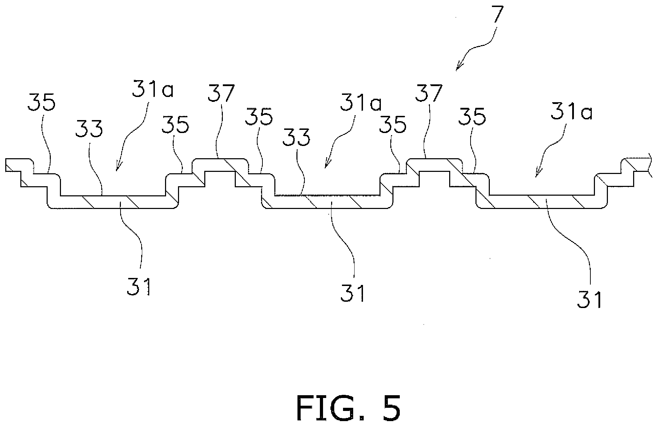

A method of manufacturing the conical-projections-sheet packaging body 1 will now be explained, with reference to FIG. 5 to FIG. 16. The plurality of manufacturing processes is explained below.

First, a blank that will constitute the conical-projections forming sheet 7 is prepared. FIG. 5 is a cross-sectional view of a blank of the conical-projections forming sheet. As is clear from the figure, the plurality of housing parts 31 is formed on the conical-projections forming sheet 7 in advance. The formation of the housing parts 31 is performed by vacuum forming, vacuum-and-pressure forming, or cold forming. For example, the plurality of recessed-shaped housing parts 31 can be formed under the following conditions: using one or more punches, a flat sheet is pressed in from an upper part of the tool die, and thereby a recessed shape is formed by the recess-shaped tool die, whose shape corresponds to the recessed part of the punch shape.

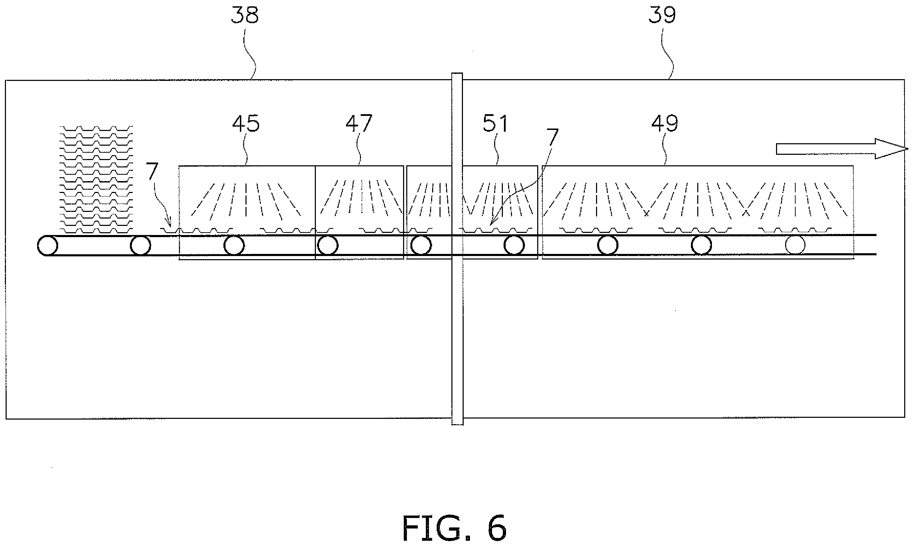

FIG. 6 is a schematic drawing that illustrates cleaning, drying, and preheating operations. As shown in the figure, a general room 38 and a clean room 39 are provided in series. A fluid chamber 45 and a rinse chamber 47 are provided in the general room 38. A drying chamber 49 is provided in the clean room 39. An air-blowing chamber 51 is provided at the boundary between the general room 38 and the clean room 39. Based on the above configuration, the conical-projections forming sheets 7 are subject to a wet-cleaning process in the general room 38 and to preheating, before drying and forming, in the clean room 39.

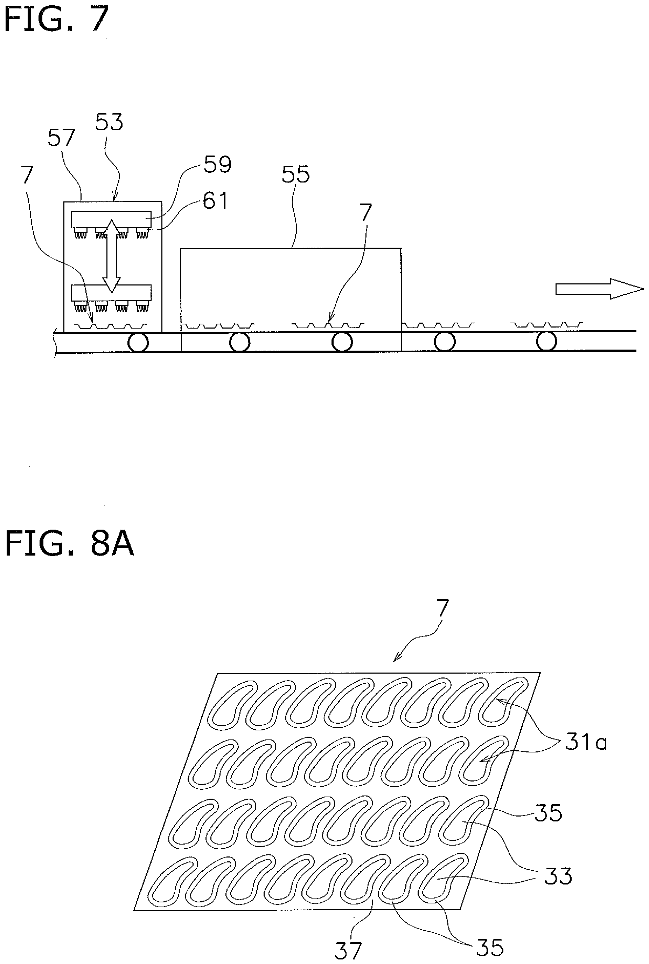

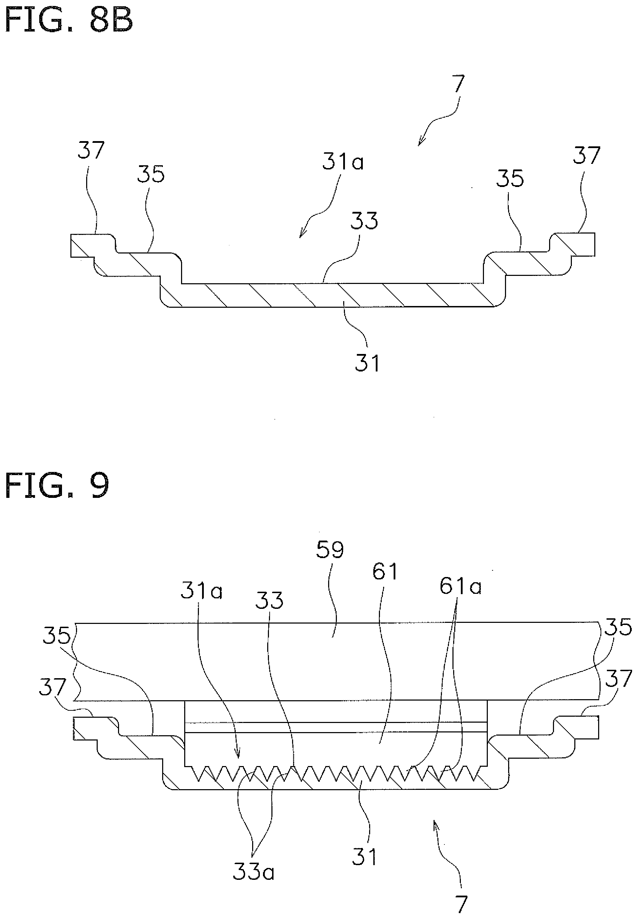

FIG. 7 is a schematic drawing that illustrates an operation of forming the conical-recess parts. As shown in the figure, a forming apparatus 53 and a cooling zone 55 are provided in series. The forming apparatus 53 is an apparatus for forming the plurality of minute conical-recess parts 33a on the bottom surfaces 33 of the conical-projections forming sheets 7. Specifically, the forming apparatus 53 includes a high-precision press 57 and a pincushion-shaped press die 59. The press die 59 has a plurality of press parts 61. As shown in FIG. 9, each press part 61 has a shape that matches the bottom surface 33 of the recessed-housing space 31 a and includes a plurality of fabricating projections 61 a on its lower surface.

As shown in FIG. 8A and FIG. 8B, each bottom surface 33 that will constitute the recessed-housing space 31a is flat prior to the forming work. FIG. 8A is a schematic drawing of the conical-projections forming sheet before the conical-recess parts are formed. FIG. 8B is a partial cross-sectional view of the conical-projections forming sheet before the conical-recess parts are formed. When the press part 61 contacts the bottom surface 33, the conical-projections forming sheet 7 has already been softened by the preheating prior to forming. As a result, as shown in FIG. 9, the plurality of conical-recess parts 33a is formed on the bottom surface 33 by the fabricating projections 61 a. FIG. 9 is a cross-sectional view for explaining the process of forming the conical-recess parts on the conical-projections forming sheet. Furthermore, the control of the press is formed by a machine capable of controlling the pressing location, the pressing pressure, the pressing time, and the like with high precision.

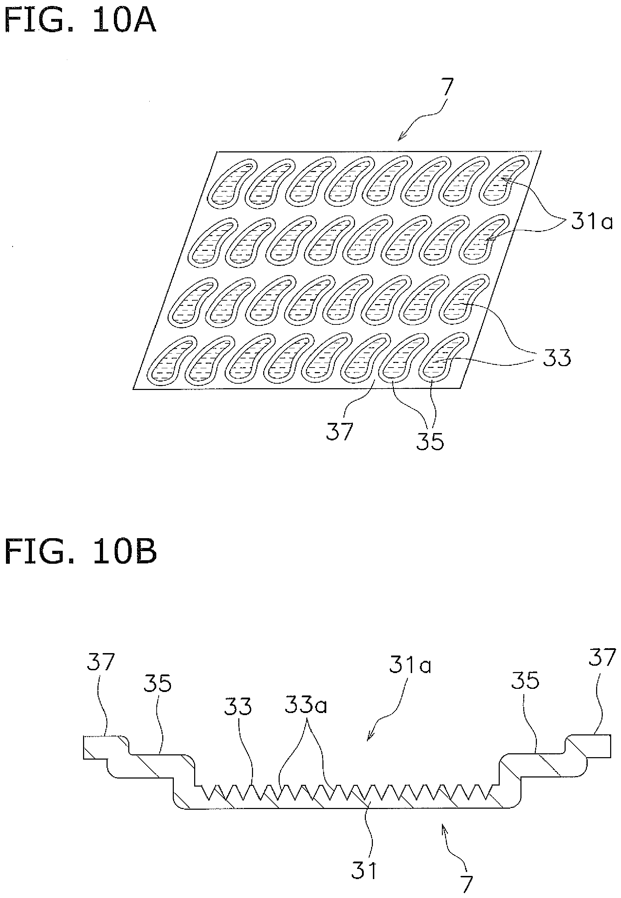

Next, the press die 59 is lifted up from the recessed-housing space 31a, whereupon, as shown in FIG. 10A and FIG. 10B, the plurality of conical-recess parts 33a is formed on the bottom surface 33 of the recessed-housing space 31 a. FIG. 10A is a schematic drawing of the conical-projections forming sheet after the conical-recess parts have been formed. FIG. 10B is a partial cross-sectional view of the conical-projections forming sheet after the conical-recess parts have been formed. As shown in the Figure, each conical-recess part 33a has, for example, a conical shape or a pyramidal shape and is open toward the upper side in the figure.

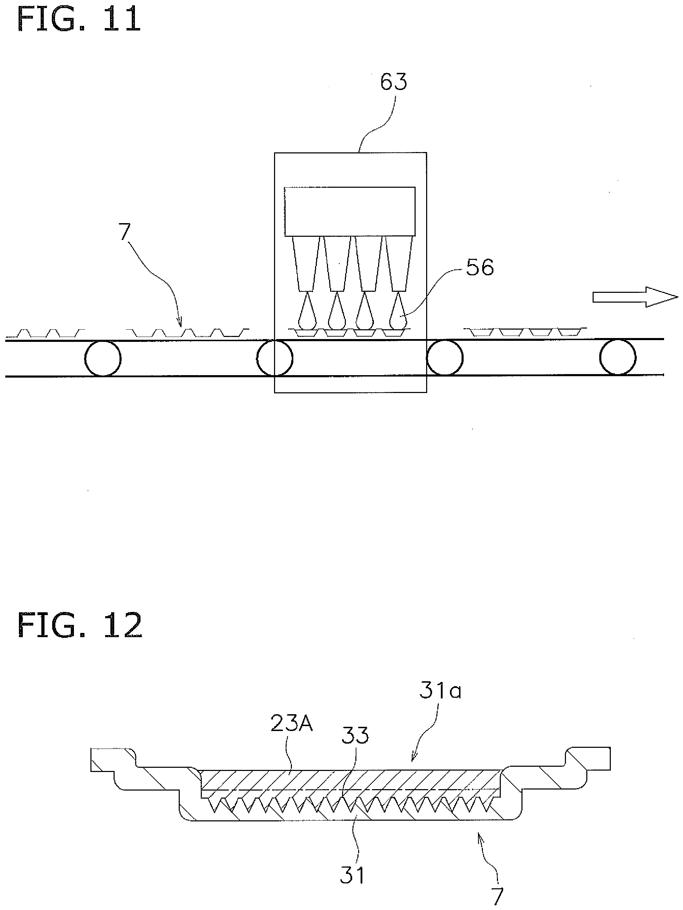

FIG. 11 is a schematic drawing for explaining a process of dripping conical-projections materials onto the conical-projections forming sheets. As shown in the figure, a dispenser 63 is provided. The dispenser 63 is an apparatus that drips the conical-projections materials onto the recessed-housing parts 31a of the housing parts 31, thereby filling the recessed-housing parts 31 a with the conical-projections materials. Conical-projections materials 56 are, for example, sodium hyaluronate dissolved in a solvent consisting of water, etc. Furthermore, a medium wherein water-soluble macromolecules in addition to water are soluble is selected as the solvent. Thus, by supplying the conical-projections material to each bottom surface 33, each bottom surface 33 is covered by a conical-projections material 23A, as shown in FIG. 12. FIG. 12 is a partial cross-sectional view of the conical-projections forming sheet filled with the conical-projections material.

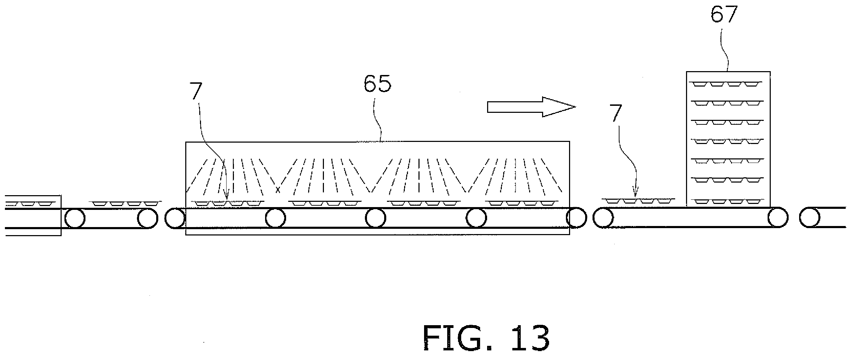

FIG. 13 is a schematic drawing for explaining a process of drying the conical-projections material. As shown in the figure, an IR-drying furnace 65 and a buffer 67 are provided. In the IR-drying furnace 65, the conical-projections materials 23A on each conical-projections forming sheet 7 are dried by heating. Thereby, moisture and the solvent agent are evaporated from the conical-projections materials 23A. As a result, starting from the state shown in FIG. 12, the conical-projections material 23A shrinks because of the drying, and the volume is reduced to the position of the chain line. Based on the above, each conical-projections sheet 23 is formed having the substrate 23a and the plurality of conical projections 23b.

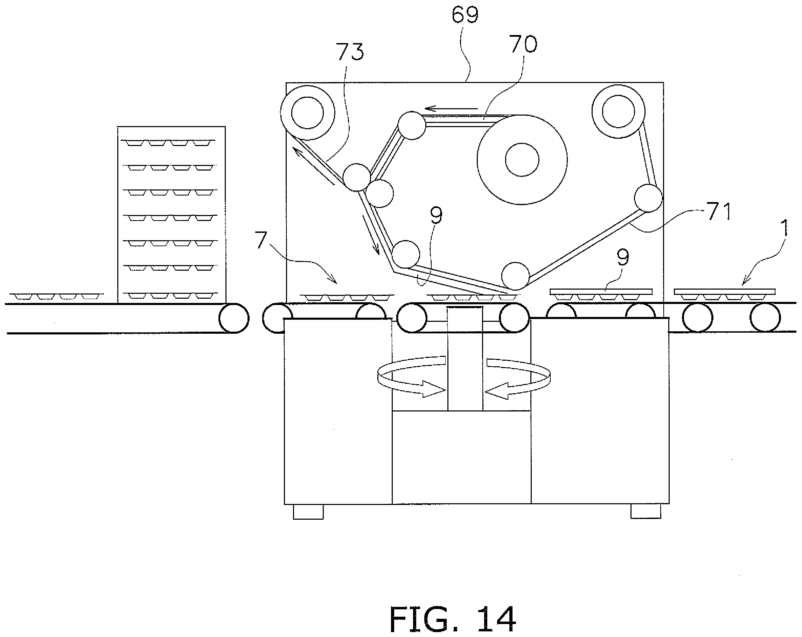

FIG. 14 is a schematic drawing for explaining a process of temporarily fixing the sealing sheets to the conical-projections forming sheets. As shown in the figure, a sealing-sheet-tacking apparatus 69 is provided. The sealing-sheet-tacking apparatus 69 is an apparatus for tacking the sealing sheets 9 to the conical-projections forming sheets 7. The sealing-sheet-tacking apparatus 69 uses a sheet material 70. The sheet material 70 includes: a base-material sheet 71; a sealing sheet 9, which is provided on the base-material sheet 71; and a separator sheet 73, which is stuck to the surface of the sealing sheet 9. The sealing-sheet-tacking apparatus 69 tacks the sealing sheets 9 to the conical-projections forming sheets 7 while peeling the separator sheet 73 from the sealing sheets 9 and meanwhile recovers the base-material sheet 71.

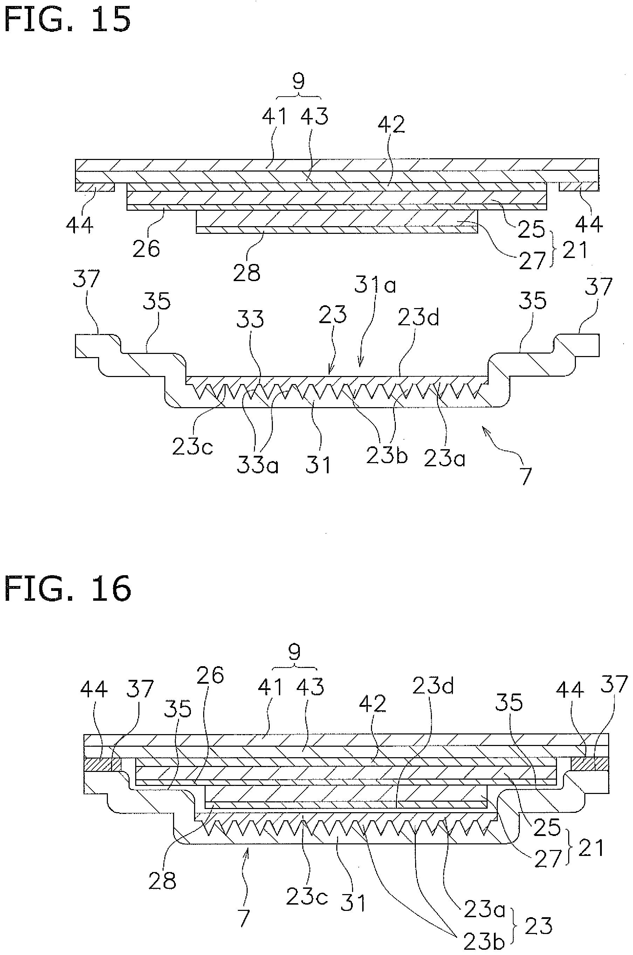

As shown in FIG. 15 and FIG. 16, a plurality of the support bodies 21 is attached beforehand to the previously discussed sealing sheets 9 at prescribed positions. That is, the support bodies 21 are attached to the conical-projections forming sheets 7 simultaneous with the tacking of the sealing sheets to the conical-projections forming sheets 7. In addition, the second bonding layer 44 is provided on each sealing sheet 9, and such is fixed to the flat surface 37 of the conical-projections forming sheet 7. FIG. 15 is a cross-sectional view of the sealing sheet and the conical-projections forming sheet. FIG. 16 is a cross-sectional view of a state wherein the sealing sheet has been temporarily fixed to the conical-projections forming sheet. In a state wherein the sealing sheet 9 is fixed to the conical-projections forming sheet 7, the support body 21 is inserted into the recessed-housing space 31 a and, as a result, is brought proximate to the conical-projections sheet 23. However, at this time, as shown in FIG. 16, a gap is maintained between the second sheet 27 of the support body 21 and the conical-projections sheet 23, and furthermore a gap is maintained between the first sheet 25 of the support body 21 and the stepped surface 35.

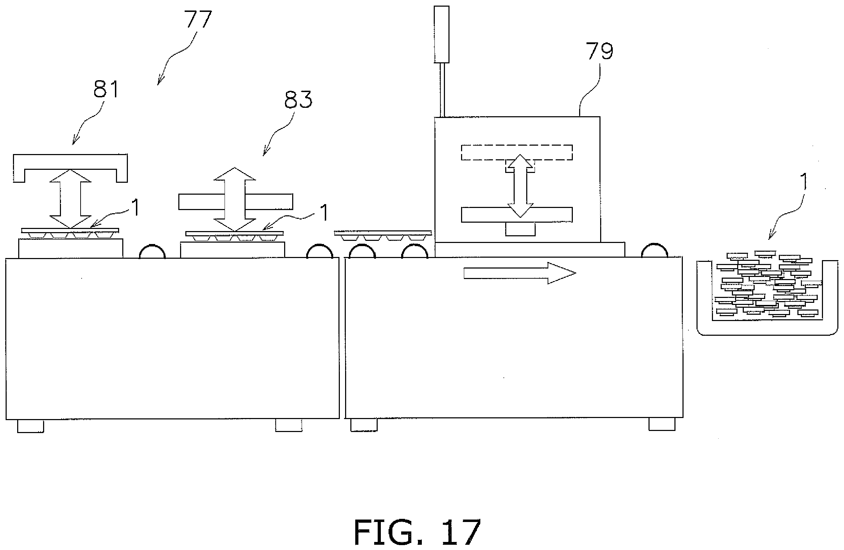

FIG. 17 is a schematic drawing for explaining a process of fixing the sealing sheets to the conical-projections forming sheets and a process of cutting up the completed conical-projections-sheet packaging bodies. As shown in the figure, a sealing apparatus 77 and a cutting apparatus 79 are provided in series. The sealing apparatus 77 includes an evacuating-and-sealing apparatus 81 and a main thermocompressing apparatus 83. The evacuating-and-sealing apparatus 81 evacuates the air from the previously mentioned gaps. The main thermocompressing apparatus 83 performs heating to bond the second sheet 27 of the support body 21 to the conical-projections sheet 23 via the first bonding layer 28 and furthermore fixes the first sheet 25 of the support body 21 to the stepped surface 35 via the first adhesive layer 26. As a result, as shown in FIG. 2, the conical-projections-sheet packaging body 1 is obtained.

Last, each conical-projections-sheet packaging body 1 is cut up into singular units by the cutting apparatus 79.

Thus, in the state wherein the conical-projections-sheet packaging body 1 has been completed, the conical-projections sheet 23 maintains a state wherein it is tightly adhered to the conical-projections forming sheet 7 via the first adhesive layer 26 of the support body 21. Consequently, the conical projections 23b are protected when the conical-projections-sheet packaging body 1 is transported or used. Specifically, the adhesive strength of the first adhesive layer 26 of the support body 21 maintains the state wherein the conical projections 23b are housed in the conical-recess parts 33a; as a result, the conical projections 23b are protected.

As discussed above, the conical-projections forming sheet 7 functions as a packaging material of the conical-projections sheet 23 but has the additional two functions below.

First, the conical-projections forming sheet 7 functions as a forming die for forming the conical projections 23b of the conical-projections sheet 23.

Second, the conical-projections forming sheet 7 functions as a protective material that protects the conical projections 23b of the conical-projections sheet 23.

As described above, the conical-projections forming sheet 7 has a plurality of functions related to the conical-projections sheet 23, and thereby there is no need to use a special member to protect the conical projections 23b, and thus the packaging is simplified. Furthermore, after the manufacture of the conical-projections sheet, procedures such as removing the conical-projections sheet from a die and further packaging the conical-projections sheet with a separate packaging member are unnecessary.

### 2. Summary of First Embodiment

### (1) Structure of the Conical-Projections-Sheet Packaging Body

The conical-projections-sheet packaging body 1 (one example of the conical-projections-sheet packaging body) includes the conical-projections sheet 23 (one example of the conical-projections sheet), the conical-projections forming sheet 7 (one example of the conical-projections forming sheet), and the sealing sheet 9 (one example of the sealing sheet). The conical-projections sheet 23 includes the substrate 23a (one example of the substrate), and the plurality of conical projections 23b (one example of the plurality of conical projections) formed on the surface 23c thereof. The conical-projections forming sheet 7 has the plurality of conical-recess parts 33a (one example of the conical-recess parts), which are used to form the plurality of conical projections 23b and wherein the conical projections 23b are disposed, and protects the conical-projections sheet 23 in the state wherein the conical-projections forming sheet 7 is tightly adhered to the surface 23c of the conical-projections sheet 23. The sealing sheet 9 is fixed to the conical-projections forming sheet 7, and thereby the conical-projections sheet 23 is sealed between the sealing sheet 9 and the conical-projections forming sheet 7.

In this case, the plurality of conical projections 23b of the conical-projections sheet 23 is formed by the plurality of conical-recess parts 33a of the conical-projections forming sheet 7; subsequently, too, the state wherein the plurality of conical projections 23b is protected by the conical-projections forming sheet 7 is maintained. Accordingly, the plurality of conical projections 23b of the conical-projections sheet 23 is reliably protected.

In addition, because the conical-projections sheet 23 is sealed between the conical-projections forming sheet 7 and the sealing sheet 9, the quality of the conical-projections sheet 23 is maintained for a long time. As discussed above, because the conical-projections forming sheet 7, together with the sealing sheet 9, is used as the member that constitutes the packaging body, the part count of the conical-projections-sheet packaging body 1 can be reduced while the plurality of conical projections 23b is reliably protected.

### (2) Method of Manufacturing the Conical-Projections-Sheet Packaging Body

The method of manufacturing the conical-projections-sheet packaging body 1 includes the following steps:
- a step that forms the plurality of conical-recess parts 33a on the conical-projections forming sheet 7 (refer to FIG. 7 to FIG. 10B);
- a step that, by supplying the conical-projections material to the conical-projections forming sheet 7, forms the conical-projections sheet 23 including the substrate 23a and the plurality of conical projections 23b, which are formed on the surface 23c thereof and disposed inside the plurality of conical-recess parts 33a (refer to FIG. 11 to FIG. 12); and
- a step that, by fixing the sealing sheet 9 to the conical-projections forming sheet 7, seals the conical-projections sheet 23 between the conical-projections forming sheet 7 and the sealing sheet 9.

In this manufacturing method, the conical-projections sheet 23 is formed in the recessed-housing parts 31 a of the conical-projections forming sheet 7, and that state is maintained. Specifically, the plurality of conical projections 23b of the conical-projections sheet 23 is formed by the plurality of conical-recess parts 33a of the conical-projections forming sheet 7; subsequently, too, the state wherein the plurality of conical projections 23b is protected by the conical-projections forming sheet 7 is maintained. Accordingly, the plurality of conical projections 23b of the conical-projections sheet 23 is reliably protected.

In addition, because the conical-projections sheet 23 is sealed between the conical-projections forming sheet 7 and the sealing sheet 9, the quality of the conical-projections sheet 23 is maintained for a long time. As discussed above, because the conical-projections forming sheet 7, together with the sealing sheet 9, is used as the member that constitutes the packaging body, the part count of the conical-projections-sheet packaging body 1 can be reduced while the plurality of conical projections 23b is reliably protected.

### 3. Other Embodiments

The above explained the first embodiment of the present invention, but the present invention is not limited to the abovementioned embodiment, and various modifications can be effected within a scope that does not depart from the gist of the invention. In particular, the embodiments and modified examples written in the present specification can be arbitrarily combined as needed.
(a) In the abovementioned embodiment, when the support bodies are attached to the conical-projections forming sheets, the support bodies have already been fixed to the sealing sheet beforehand and are attached to the conical-projections forming sheets together with the sealing sheet. However, the support bodies may be prepared separately from the sealing sheet and attached one at a time to the conical-projections forming sheets.
(b) In the abovementioned embodiment, each support body includes two sheets (the first sheet 25 and the second sheet 27), but the support body may include one sheet.
(c) In the abovementioned embodiment, each support body 21 includes the adhesive layer, which covers the entire surface of the conical-projections sheet 23 and further extends entirely around the outer-perimeter side; however, the shape of the support body, the positional relationship with the conical-projections sheet, the shape of the adhesive layer, and the like can be modified as appropriate.
(d) In the abovementioned embodiment, the support-body-attached, conical-projections sheet is peeled, together with the sealing sheet, from the conical-projections forming sheet; however, a configuration may be adopted wherein the support-body-attached, conical-projections sheet is peeled from the conical-projections forming sheet after the sealing sheet has been peeled.
(e) In the abovementioned embodiment, the location at which the plurality of conical-recess parts 33a is formed on the conical-projections forming sheet 7 is the bottom surface 33 of the recessed-housing space 31 a; however, the plurality of conical-recess parts may be formed in a portion other than a recessed part. For example, the conical-projections forming sheet may be a flat member, and the plurality of conical-recess parts may be formed on the surface thereof.
(f) In the abovementioned embodiment, the conical-projections sheet 23 is tightly adhered to the bottom surface 33 by fixing the support body 21 to the conical-projections forming sheet 7 via the first adhesive layer 26, and thereby the state wherein the conical projections 23b are protected by the conical-recess parts 33a is maintained; however, the conical-projections sheet may be tightly adhered to the bottom surface by some other means, or both may be tightly adhered to one another without devising any special means.

### 4. Second Embodiment

A second embodiment is explained below. The basic configuration and the manufacturing method of the second embodiment are the same as those of the first embodiment. Accordingly, the explanation below focuses only on points of difference.

### (1) Conical-Projections-Sheet Packaging Body

The conical-projections-sheet packaging body 1 is substantially the same as that of the first embodiment.

### (1-1) Support-Body-Attached, Conical-Projections Sheet

The support-body-attached, conical-projections sheet 3 is substantially the same as that of the first embodiment.

The first adhesive layer 26 consists of, for example, an acrylic component.

### (1-2) Packaging Material

The packaging material 5 is substantially the same as that of the first embodiment.

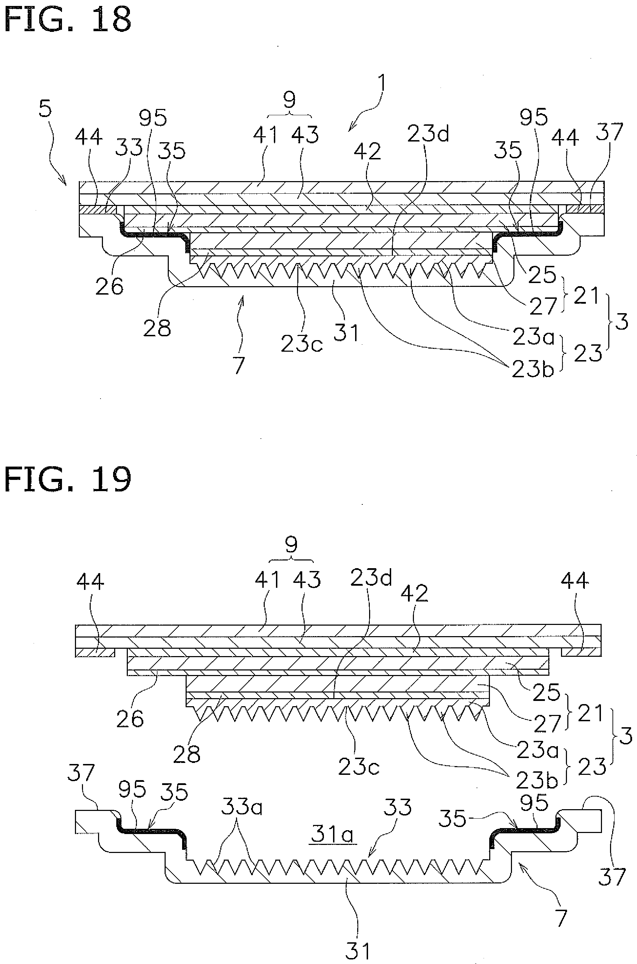

As shown in FIG. 18 and FIG. 19, the stepped surface 35 is coated with a die-releasing agent 95 to make it easy for the outer-perimeter part of the support body 21 to be peeled off. The die-releasing agent 95 consists of, for example, silicone. The die-releasing agent 95 preferably includes a material that does not damage the human body.

Furthermore, in the present embodiment, the die-releasing agent 95 is formed also on a tubular surface (the surface connected to the bottom surface 33) located on the inner-perimeter side of the stepped surface 35 and on a tubular surface (the surface connected to the flat surface 37) located on the outer-perimeter side of the stepped surface 35. However, with an aim to avoid contact with the conical-projections sheet 23, the die-releasing agent 95 is not applied to the bottom surface 33.

Taking into consideration that the conical-projections forming sheet 7 is used as a member whereon the plurality of conical-recess parts 33a is to be formed, the conical-projections forming sheet 7 has a monolayer structure prepared with a polyolefin based resin such as polyethylene, polypropylene, or the like. However, the conical-projections forming sheet 7 may include a plurality of layers.

The plurality of conical-recess parts 33a, which is for forming and housing the conical projections 23b of the conical-projections sheet 23, is formed on the bottom surface 33 of the conical-projections forming sheet 7.

The support-body-attached, conical-projections sheet 3 is housed in the recessed-housing space 31 a of the housing part 31. Specifically, the conical-projections sheet 23 is formed in the state wherein it is tightly adhered to the bottom surface 33 and thereby the bottom surface 33 is in the state wherein it is tightly adhered to the surface 23c of the substrate 23a of the conical-projections sheet 23. In this state, the conical projections 23b are disposed inside the conical-recess parts 33a. Based on the above, the bottom surface 33 functions as a formation surface and a protective surface of the conical projections 23b.

In addition, as discussed above, by virtue of the support body 21 being adhered to the conical-projections forming sheet 7, the state is maintained wherein the plurality of conical projections 23b of the conical-projections sheet 23 is disposed in the plurality of conical-recess parts 33a of the conical-projections forming sheet 7. Furthermore, when the support-body-attached, conical-projections sheet 3 is removed from the conical-projections forming sheet 7, the outer-perimeter part of the support body 21 (the portion fixed by the first adhesive layer 26) can be easily peeled from the conical-projections forming sheet 7. Furthermore, the first adhesive layer 26 functions as an adhesive portion after the support-body-attached, conical-projections sheet 3 has been taken out.

Furthermore, as discussed above, because the stepped surface 35 is coated with the die-releasing agent 95 as the process of reducing the peel strength imparted to the portion of the conical-projections forming sheet 7 at which the support body 21 is adhered, the outer-perimeter part of the support body 21 (the portion fixed by the first adhesive layer 26) can easily be peeled from the conical-projections forming sheet 7. Specifically, the peel strength between the support body 21 and the conical-projections forming sheet 7 is 0.5-4.0 (N/10 mm) and preferably is 0.5-2.0 (N/10 mm).

The sealing sheet 9 is a sheet-shaped member that is stuck to the inner-side surface of the conical-projections forming sheet 7 in order to seal the support-body-attached, conical-projections sheet 3 inside the recessed-housing space 31 a between the sealing sheet 9 and the conical-projections forming sheet 7. Specifically, the sealing sheet 9 includes the surface-resin layer 41 and the metal layer 43.

The surface-resin layer 41 is capable of being printed upon and further functions to protect the metal layer 43.

The metal layer 43 is provided on a lower surface of the surface-resin layer 41. The metal layer 43 includes aluminum or an alloy thereof and has a high degree of moisture proofness.

The second adhesive layer 42 and the second bonding layer 44 are provided on the surface of the metal layer 43 on the side opposite the surface-resin layer 41. The second adhesive layer 42 is for the purpose of adhering the support body 21 to the sealing sheet 9. The second adhesive layer 42 consists of, for example, an acrylic-based component. The second bonding layer 44 is for the purpose of bonding the sealing sheet 9 to the conical-projections forming sheet 7; specifically, the second bonding layer 44 is bonded to the flat surface 37 of the conical-projections forming sheet 7.

Furthermore, when the sealing sheet 9 is peeled from the conical-projections forming sheet 7, the peel strength between the sealing sheet 9 and the support body 21 maintains the adhesion state between the sealing sheet 9 and the support body 21; however, when the support body 21 is peeled from the sealing sheet 9, it is necessary that it be easily peelable. Specifically, the peel strength between the sealing sheet 9 and the support body 21 is 2.0-6.5 (N/10 mm) and preferably is 3.0-5.0 (N/mm).

Thus, the sealing sheet 9 is stuck to the inner-side surface of the conical-projections forming sheet 7 in a state wherein the sealing sheet 9 covers the conical-projections sheet 23, and thereby both seal the recessed-housing space 31 a.

In the conical-projections-sheet packaging body 1 discussed above, the conical projections 23b of each conical-projections sheet 23 are formed by the plurality of conical-recess parts 33a of the conical-projections forming sheet 7 (discussed below) and subsequently are also protected by the conical-projections forming sheet 7.

As shown in FIG. 19, when removing the support-body-attached, conical-projections sheet 3 from the conical-projections-sheet packaging body 1, a user peels the sealing sheet 9 from the conical-projections forming sheet 7. FIG. 19 is a cross-sectional view that shows a state wherein the sealing sheet has been peeled from the conical-projections forming sheet. At this time, as shown in FIG. 19, the support-body-attached, conical-projections sheet 3, together with the sealing sheet 9, is separated from the conical-projections forming sheet 7. The support-body-attached, conical-projections sheet 3 is removed from the recessed-housing parts 31a of the conical-projections forming sheet 7 by the aforementioned operation.

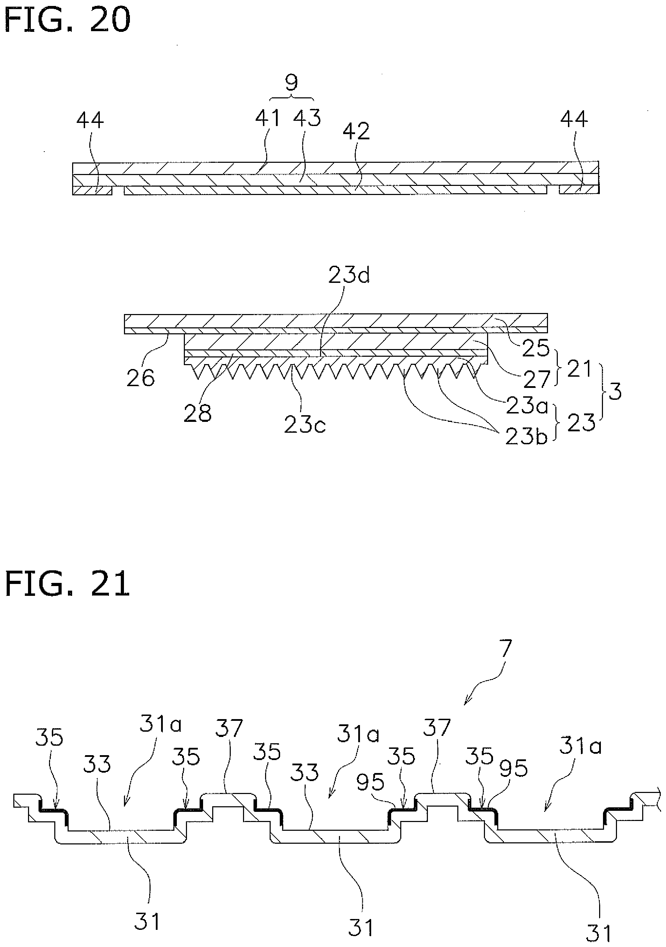

Next, as shown in FIG. 20, the user peels the support-body-attached, conical-projections sheet 3 from the sealing sheet 9. FIG. 20 is a cross-sectional view that shows a state wherein the support-body-attached, conical-projections sheet has been peeled from the sealing sheet. As a result, the user becomes able to use the support-body-attached, conical-projections sheet 3 in accordance with its purpose. In the case of a transdermal patch, the support-body-attached, conical-projections sheet 3 is stuck to the skin of a person.

In the above operation, as shown in FIG. 18 and FIG. 19, the portion of the conical-projections forming sheet 7 at which the support body 21 is adhered is subject to the process of reducing the peel strength, and therefore the support body 21, together with the conical-projections sheet 23, easily peels from the conical-projections forming sheet 7 without reducing the strength with which the support body 21 adheres to the human body or other members. Accordingly, when the conical-projections sheet 23 is removed from the conical-projections forming sheet 7 and used, the plurality of conical projections 23b tends not to get damaged.

### (2) Method of Manufacturing the Conical-Projections-Sheet Packaging Body

A method of manufacturing the conical-projections-sheet packaging body 1 is substantially the same as that of the first embodiment. Furthermore, FIG. 9B, FIG. 10, FIG. 11B, FIG. 13, FIG. 16, and FIG. 17 are attached as the drawings corresponding to the present embodiment.

First, a blank that will constitute the conical-projections forming sheet 7 is prepared. FIG. 21 is a cross-sectional view of a blank of the conical-projections forming sheet. As is clear from the figure, the plurality of housing parts 31 is formed on the conical-projections forming sheet 7 in advance. The formation of the housing parts 31 is performed by vacuum forming, vacuum-and-pressure forming, or cold forming. For example, the plurality of recessed-shaped housing parts 31 can be formed under the following conditions: using one or more punches, a flat sheet is pressed in from an upper part of the tool die, and thereby a recessed shape is formed by the recess-shaped tool die, whose shape corresponds to the recessed part of the punch shape.

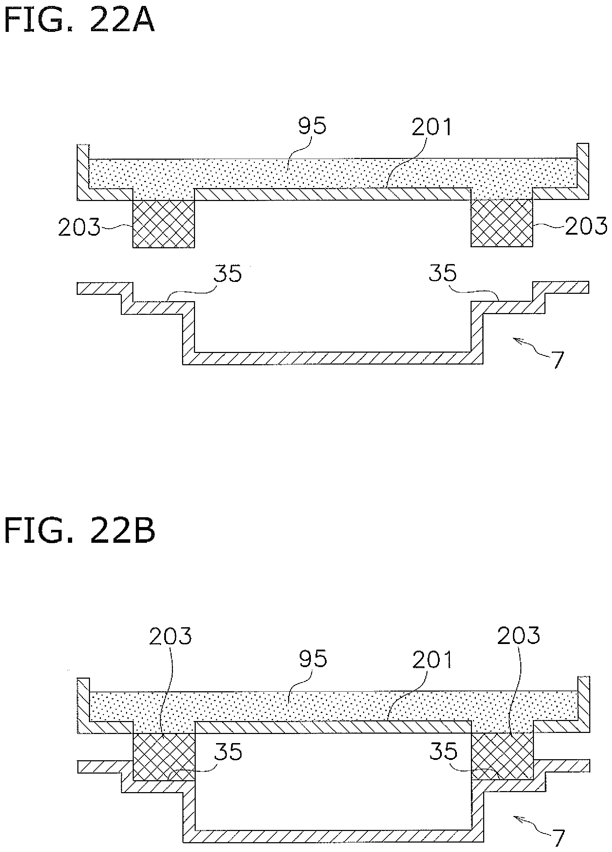

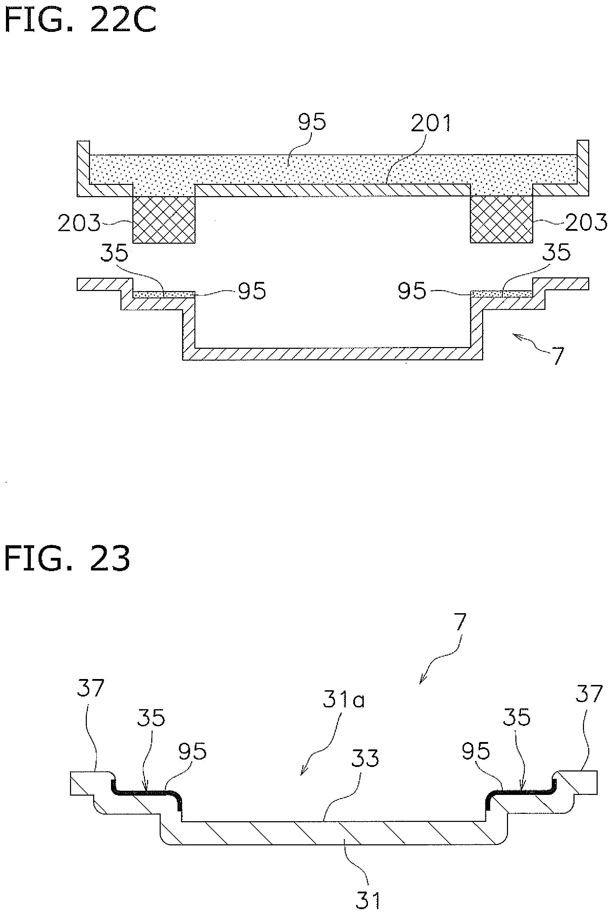

Furthermore, as shown in FIG. 21, each stepped surface 35 is coated with the die-releasing agent 95. The method of applying the die-releasing agent 95 will now be explained, with reference to FIG. 22A to FIG. 22C. FIG. 22A to FIG. 22C are schematic drawings that show a process of coating the stepped surface with the die-releasing agent. As shown in FIG. 22A, a tank 201, wherein the liquid die-releasing agent 95 is stored, is prepared. A liquid-supplying member 203, whose shape corresponds to that of the stepped surface 35, is provided on the lower surface of the tank 201. The liquid-supplying member 203 is, for example, made of felt and is a member made capable of oozing the die-releasing agent 95 out from a lower part of the tank 201. Accordingly, as shown in FIG. 22B, the tank 201 is lowered and, when the liquid-supplying member 203 makes contact with the stepped surface 35, the die-releasing agent 95 is supplied to the stepped surface 35. As a result, as shown in FIG. 22C, when the tank 201 is raised, the state remains wherein the stepped surface 35 is coated with the die-releasing agent 95. In addition, a spraying method is another method of applying the die-releasing agent 95.

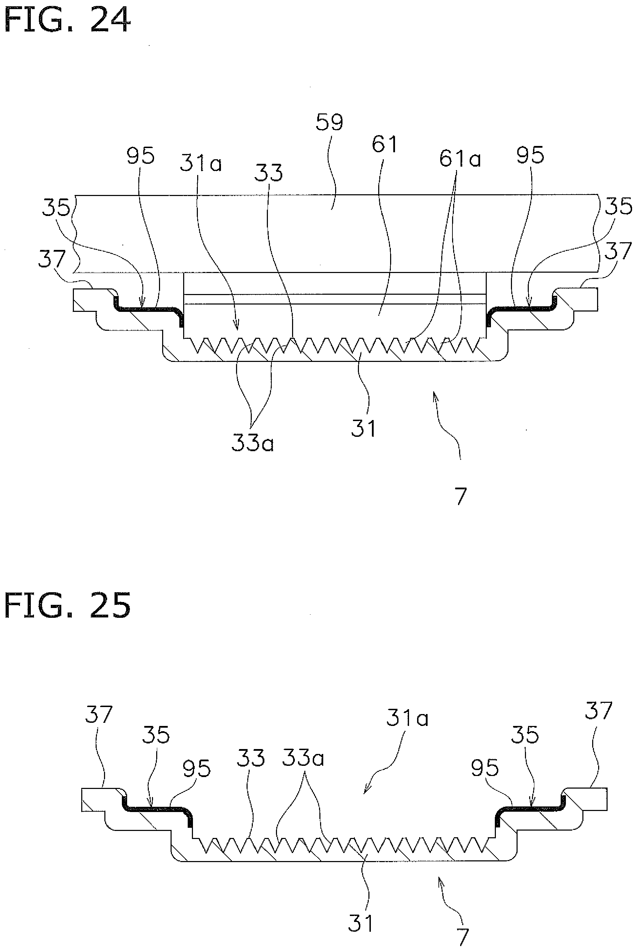

FIG. 23 is a partial cross-sectional view of the conical-projections forming sheet before the conical-recess parts have been formed. When the press part 61 contacts the bottom surface 33, the conical-projections forming sheet 7 has already been softened by the preheating prior to forming. As a result, as shown in FIG. 24, the plurality of conical-recess parts 33a is formed on the bottom surface 33 by the fabricating projections 61 a. FIG. 24 is a cross-sectional view for explaining a process of forming the conical-recess parts on the conical-projections forming sheet. Furthermore, the control of the press is performed by a machine capable of controlling the pressing location, the pressing pressure, the pressing time, and the like with high precision.

FIG. 25 is a partial cross-sectional view of the conical-projections forming sheet after the conical-recess parts have been formed. As shown in the figure, each conical-recess part 33a has, for example, a conical shape or a pyramidal shape and is open toward the upper side in the figure.

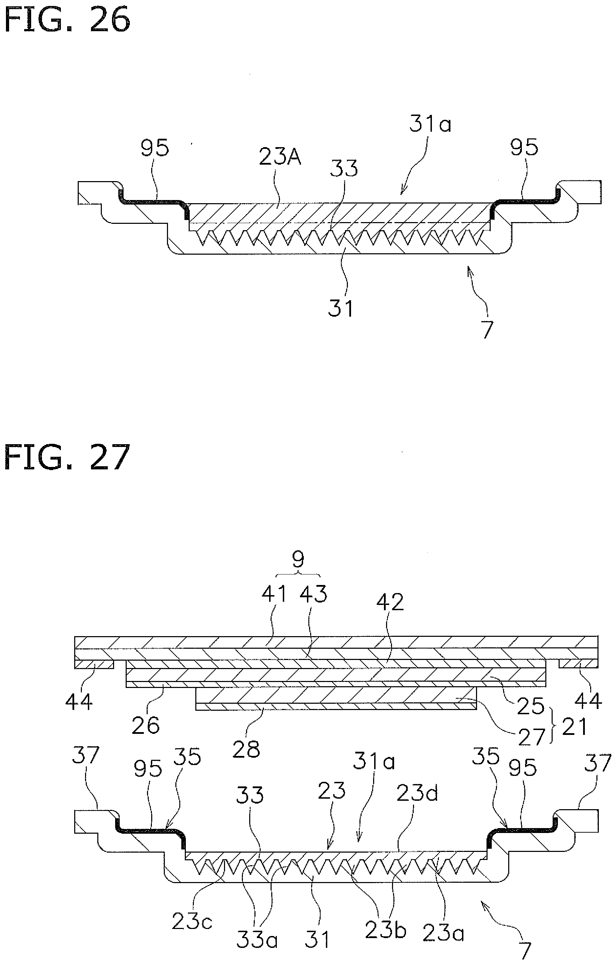

As shown in FIG. 26, the bottom surface 33 is covered by the conical-projections material 23A. FIG. 26 is a partial cross-sectional view of a conical-projections forming sheet filled with the conical-projections material.

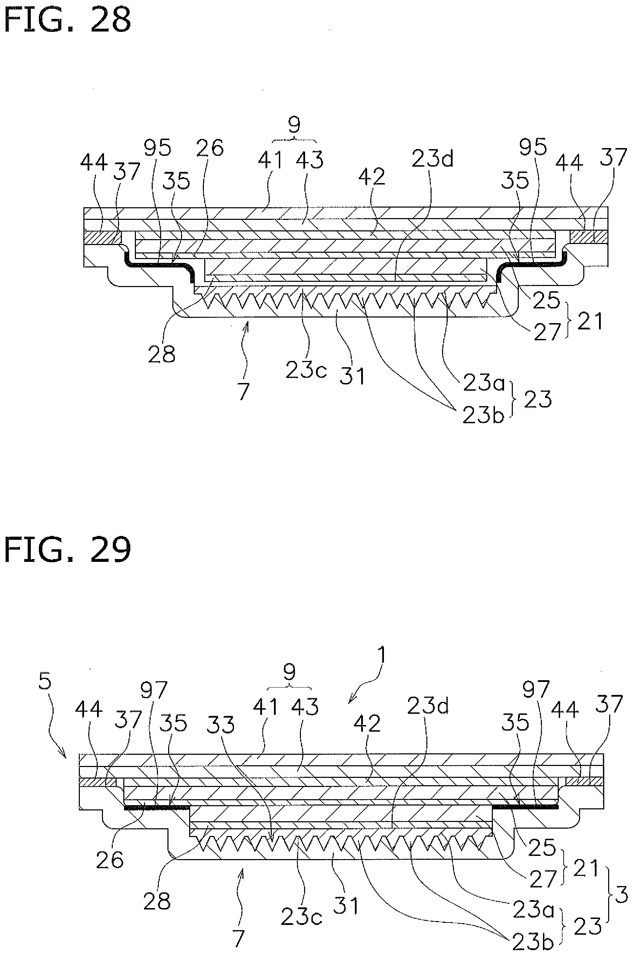

As shown in FIG. 27 and FIG. 28, a plurality of the support bodies 21 is attached beforehand to the previously discussed sealing sheets 9 at prescribed positions. That is, the support bodies 21 are attached to the conical-projections forming sheets 7 simultaneous with the tacking of the sealing sheets 9 to the conical-projections forming sheets 7. In addition, the second bonding layer 44 is provided on each sealing sheet 9, and such is fixed to the flat surface 37 of the conical-projections forming sheet 7. FIG. 27 is a cross-sectional view of the sealing sheet and the conical-projections forming sheet. FIG. 28 is a cross-sectional view of a state wherein the sealing sheet has been temporarily fixed to the conical-projections forming sheet. In a state wherein the sealing sheet 9 is fixed to the conical-projections forming sheet 7, the support body 21 is inserted into the recessed-housing space 31 a and, as a result, is brought proximate to the conical-projections sheet 23. However, at this time, as shown in FIG. 28, the gap is maintained between the second sheet 27 of the support body 21 and the conical-projections sheet 23, and furthermore the gap is maintained between the first sheet 25 of the support body 21 and the stepped surface 35.

### 5. Third Embodiment

In the second embodiment, the die-releasing agent is applied to the stepped surface 35 as well as the tubular surface located on both sides in the radial direction thereof, but the die-releasing agent may be formed only at the portion at which the support body 21 is adhered.

The third embodiment is explained below with reference to FIG. 29. FIG. 29 is a cross-sectional view of the conical-projections-sheet packaging body according to the third embodiment.

In the present embodiment, a die-releasing agent 97 is applied only to the stepped surface 35. Furthermore, there is no need for the entire stepped surface 35 to be coated with the die-releasing agent, and only the portion at which the support body 21 is adhered should be coated.

### 6. Fourth Embodiment

In the second embodiment, the die-releasing agent is applied as the process of reducing the peel strength provided at the portion of the conical-projections forming sheet at which the support body is adhered, but the peel strength may be reduced by some other means.

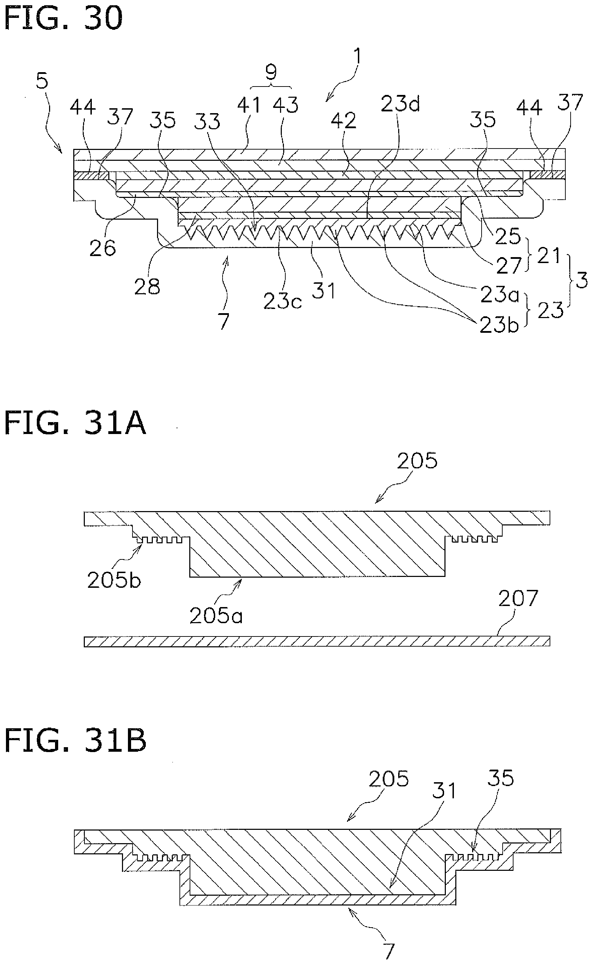

The fourth embodiment is explained below, with reference to FIG. 30. FIG. 30 is a cross-sectional view of the conical-projections-sheet packaging body according to the fourth embodiment.

In the present embodiment, the die-releasing agent is not applied to the stepped surface 35; instead, a material for improving die-releasing characteristics is employed on the conical-projections forming sheet 7. Examples of such a material include polyethylene (PE) resin, polypropylene (PP) resin, polyethylene terephthalate (PET), and polystyrene (PS), which exhibit a die-releasing effect. These are increasingly preferable in the order from the former resin to the latter resin.

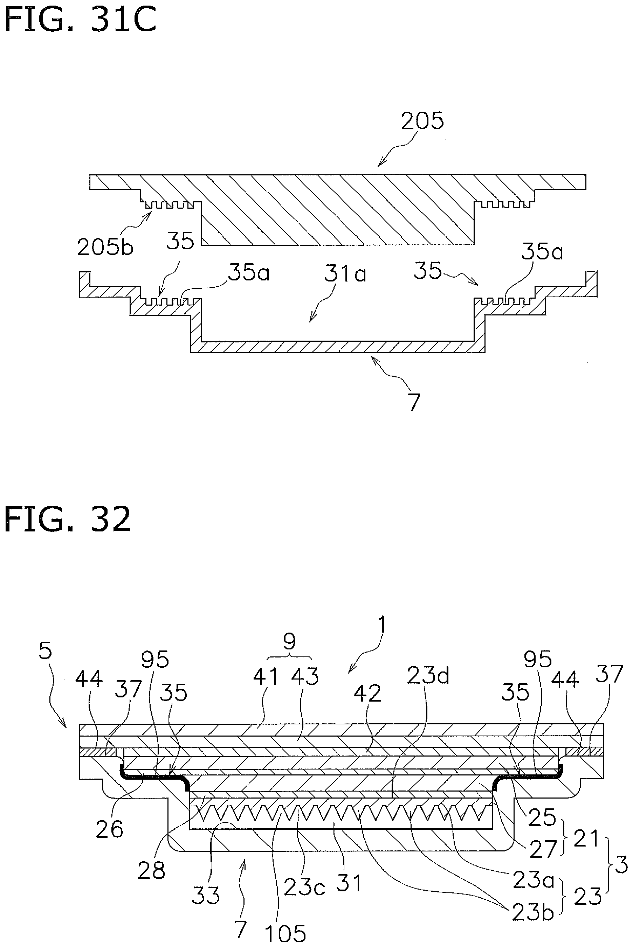

Furthermore, to increase the die-releasing effect, the contact-surface area of the adhesive agent can be reduced by embossing the stepped surface 35. A working example wherein the stepped surface 35 is embossed will now be explained, with reference to FIG. 31A to FIG. 31C. FIG. 31A to FIG. 31C are schematic drawings that show a process of embossing the stepped surface. As shown in FIG. 31A to FIG. 31C, a forming die 205 includes a protruding part 205a, which corresponds to the recessed-housing space 31a, and an embossing part 205b, which corresponds to the stepped surface 35. When the forming die 205 is pressed against a tray resin material 207, as shown in FIG. 31B, the recessed-housing space 31 a and the stepped surface 35 are formed. In addition, the stepped surface 35 is embossed. As a result, as shown in FIG. 31C, an embossed surface 35a is formed on the stepped surface 35.

Furthermore, on top of using a material having the die-releasing effect, it is also possible to apply a die-releasing agent. In such a case, the die-releasing effect will be further increased.

### 7. Fifth Embodiment

In the second embodiment, the conical-projections sheet 23 is formed using the conical-projections forming sheet 7 as a mold, but the conical-projections sheet 23 may be disposed in the recessed-housing parts 31 a of the conical-projections forming sheet 7 after the conical-projections sheet 23 has been manufactured by some other apparatus.

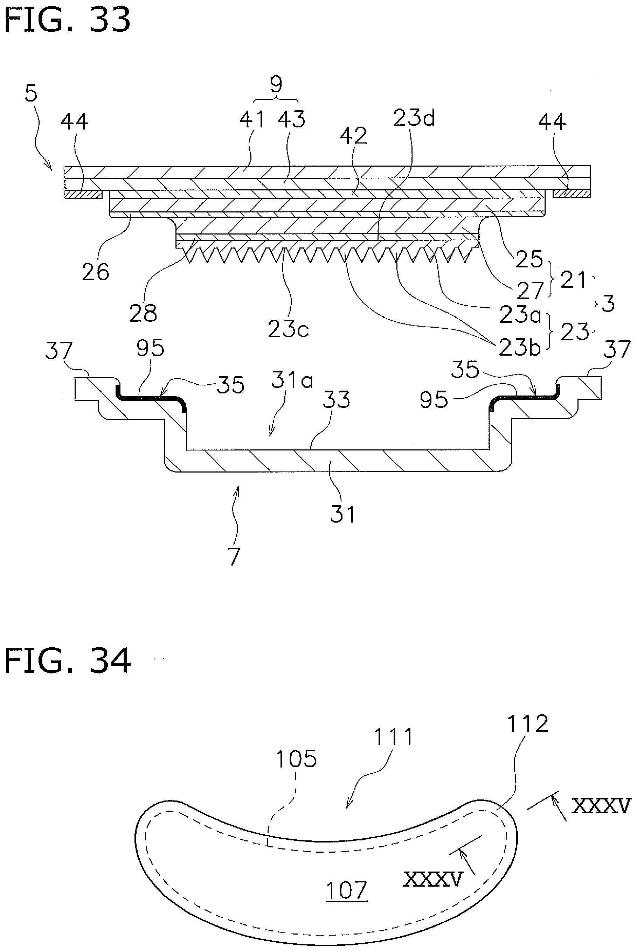

The fifth embodiment will be explained below, referencing FIG. 32 and FIG. 33. FIG. 32 is a cross-sectional view of the conical-projections-sheet packaging body according to the fifth embodiment. FIG. 33 is a schematic drawing for explaining, according to the fifth embodiment, a process of temporarily fixing the sealing sheet to the conical-projections forming sheet.

In the present embodiment, the bottom surface 33 of the conical-projections forming sheet 7 is a flat surface, and a space 10 is maintained between the bottom surface 33 and the plurality of conical projections 23b of the conical-projections sheet 23. That is, the conical-projections sheet 23 is maintained at a position spaced apart from the bottom surface 33 in the state wherein the conical-projections sheet 23 is supported by the support body 21. Thus, the conical projections 23b tend not to come into contact with other members.

An operation of bonding the sealing sheet 9 to the conical-projections forming sheet 7 will now be explained. At this time, as shown in FIG. 32, the support body 21 and the conical-projections sheet 23 (that is, the support-body-attached, conical-projections sheet 3) has already been attached to the sealing sheet 9 beforehand. In this state, when the sealing sheet 9 is stuck to the conical-projections forming sheet 7, the conical-projections sheet 23 is disposed in the recessed-housing parts 31 a of the conical-projections forming sheet 7.

### 8. Summary of Second Embodiment

### (1) Structure of Conical-Projections-Sheet Packaging Body

The conical-projections-sheet packaging body 1 (one example of the conical-projections-sheet packaging body) includes the conical-projections sheet 23 (one example of the conical-projections sheet), the conical-projections forming sheet 7 (one example of the conical-projections forming sheet), and the support body 21 (one example of the support body). The conical-projections sheet 23 includes the substrate 23a (one example of the substrate) and the plurality of conical projections 23b (one example of the conical projections) formed on the surface of the substrate 23a. The conical-projections forming sheet 7 has the recessed-housing parts 31 a (one example of the recessed-housing parts) wherein the conical-projections sheet 23 is housed. The support body 21 is fixed to a second surface of the substrate 23a of the conical-projections sheet 23 and is further adhered to the conical-projections forming sheet 7. At the portion of the conical-projections forming sheet 7 at which the support body 21 is adhered, the die-releasing agents 95, 97 are applied and a material that exhibits the die-releasing effect is used (one example of processes for reducing the peel strength).

In this case, because the processes for reducing the peel strength are performed at the portion of the conical-projections forming sheet 7 at which the support body 21 is adhered, the support body 21, together with the conical-projections sheet 23, becomes easy to peel from the conical-projections forming sheet 7 without reducing the strength with which the support body 21 adheres to the human body or other members. Accordingly, when the conical-projections sheet 23 is removed and used, the plurality of conical projections 23b tends not to get damaged.

### (2) Method of Manufacturing the Conical-Projections-Sheet Packaging Body

The method of manufacturing the conical-projections-sheet packaging body 1 (one example of the conical-projections-sheet packaging body) includes the following steps:
- a step that adheres the support body 21 (one example of the support body) to the sealing sheet 9 (one example of the sealing sheet);
- a step that, at the portion of the conical-projections forming sheet 7 (one example of the conical-projections forming sheet) at which the support body 21 is adhered, the die-releasing agents 95, 97 are applied and a material that exhibits the die-releasing effect is used (one example of processes for reducing the peel strength);
- a step that disposes the conical-projections sheet 23 (one example of the conical-projections sheet) in the recessed-housing parts 31 a (one example of the recessed-housing parts) of the conical-projections forming sheet 7;
- a step that sticks the sealing sheet 9 to the conical-projections forming sheet 7;
- a step that fixes the conical-projections sheet 23 to the support body 21; and
- a step that adheres the support body 21 to the conical-projections forming sheet 7.

In this case, because the processes for reducing the peel strength are performed at the portion of the conical-projections forming sheet 7 at which the support body 21 is adhered, the support body 21, together with the conical-projections sheet 23, is easy to peel from the conical-projections forming sheet 7 without reducing the strength with which the support body 21 adheres to the human body or other members. Accordingly, when the conical-projections sheet 23 is removed from the conical-projections forming sheet 7 and used, the plurality of conical projections 23b tends not to get damaged.

Furthermore, unless particularly stated, the order of the steps is not limited. In addition, the steps may be performed simultaneously or partially overlapping.

### 9. Other Embodiments

The above explained the second to fifth embodiments of the present invention, but the present invention is not limited to the abovementioned embodiment, and various modifications can be effected within a scope that does not depart from the gist of the invention. In particular, the embodiments and modified examples written in the present specification can be arbitrarily combined as needed.
(a) In the second embodiment, when the support bodies 21 are attached to the conical-projections forming sheets 7, the support bodies 21 have already been fixed to the sealing sheet 9 beforehand and are attached to the conical-projections forming sheets 7 together with the sealing sheet 9. However, the support bodies may be prepared separately from the sealing sheet and attached one at a time to the conical-projections forming sheets.
(b) In the second embodiment, each support body 21 includes two sheets (the first sheet 25 and the second sheet 27), but the support body may include one sheet.
(c) In the second embodiment, each support body 21 includes the first adhesive layer 26, which covers the entire surface of the conical-projections sheet 23 and further extends entirely around the outer-perimeter side; however, the shape of the support body, the positional relationship with the conical-projections sheet, the shape of the adhesive layer, and the like can be modified as appropriate.
(d) In the second embodiment, the time at which the die-releasing agent 95 is applied is after the completion of the blank of the conical-projections forming sheet 7, but the time at which the die-releasing agent 95 is applied is not particularly limited.

### 10. Sixth Embodiment

As a modified example of the first embodiment, an embodiment will be explained wherein the conical-projections forming sheet is a flat member and the plurality of conical-recess parts is formed on the surface thereof.

### (1) Conical-Projections-Sheet Packaging Body

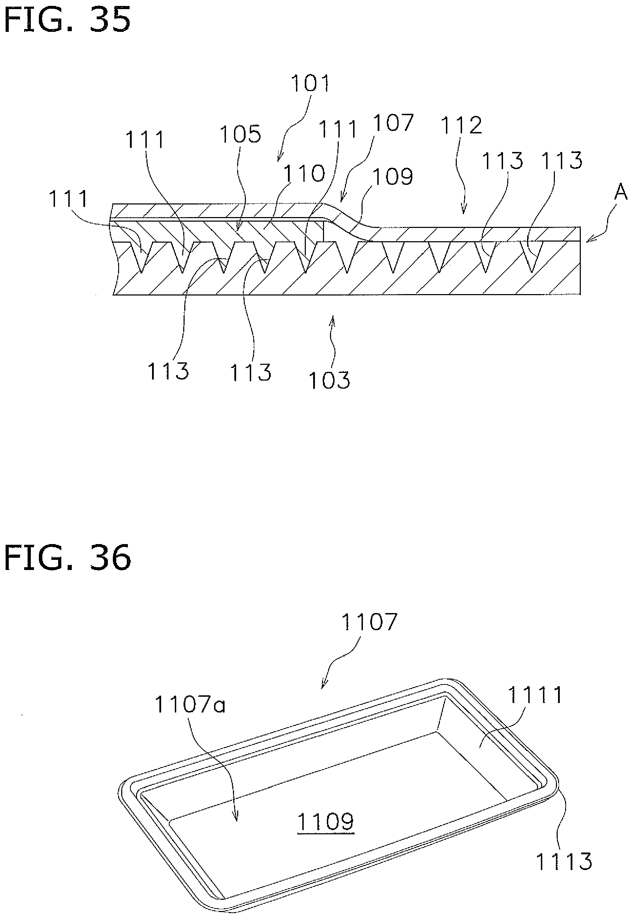

The conical-projections-sheet packaging body 1 according to the sixth embodiment will now be explained, with reference to FIG. 34 and FIG. 35. FIG. 34 is a plan view of the conical-projections-sheet packaging body according to the sixth embodiment of the present invention. FIG. 35 is a cross-sectional view taken along line XXXV-XXXV in FIG. 34 and is a cross-sectional view of the conical-projections-sheet packaging body.

A conical-projections-sheet packaging body 101 includes a conical-projections sheet 105, a microneedle-forming sheet 103, and a sealing sheet 107. The forming sheet 103 and the sealing sheet 107 are primary packaging materials that package the conical-projections sheet 105. Thus, the packaging body 101 is a primary package, the shape of which is a flat, plate-shaped piece. Accordingly, the degrees of freedom of a subsequent secondary package are increased. For example, a vacuum package and a design package are possible.

Furthermore, in the embodiment below, the conical-projections sheet housed in the primary packaging material is singular, but a plurality of the conical-projections sheets may be housed.

### (1-1) Conical-Projections Sheet

The conical-projections sheets 105 are members that are packaged by the primary packaging material, which was discussed above, and are used by being removed from the primary packaging material as needed.

The conical-projections sheet 105 is a sheet-shaped article whereon a plurality of microneedles 111 are formed on one surface thereof. The thickness of the conical-projections sheet 105 is approximately several hundred micrometers. The overall planar shape of the conical-projections sheet 105 is smoothly curved and has a crescent-moon shape, as shown in FIG. 34. The shape of the conical-projections sheet 105 may be a circle, an ellipse, a triangle, a quadrangle, a square, a magatama (comma), or some other shape. If the conical-projections sheet 105 is, for example, a quadrangle, then one side is approximately several to several tens of millimeters.

The structure of the conical-projections sheet 105 will now be explained further, with reference to FIG. 34. The conical-projections sheet 105 includes a sheet-shaped substrate 110 and the plurality of microneedles 111. The plurality of microneedles 111 is formed on a lower surface (one example of a first surface) of the substrate 110. Each microneedle 111 has, for example, a conical shape or a pyramidal shape with a height of 10-1000 µm, wherein the ratio of the cross-sectional diameter, at the base, to the height is 1:0.2-1:5 and the aspect ratio (height/cross-sectional diameter) is high.

The conical-projections sheet 105 is a transdermal patch that is used, for example, to administer a drug or the like by affixing the patch such that it contacts the skin of a person. Specifically, the administration of the drug or the like is promoted by the substrate 110 being stuck onto the skin and the microneedles 111 piercing the skin.

The conical-projections sheet 105 consists of, as the principal material, for example, a water-soluble drug, or, a water-soluble macromolecule, such as hyaluronate, a water-soluble collagen, dextran, chondroitin sulfate, or the like, to which a drug is added. Furthermore, the water-soluble macromolecule to which a pharmaceutical agent has been added is preferably an in-vivo-soluble, water-soluble macromolecule, for example, an in-vivo-soluble, water-soluble macromolecule such as sodium chondroitin sulfate, hyaluronate, or dextran.

Furthermore, the water-soluble macromolecule is a substance of at least 1 selected from the group consisting of sodium chondroitin sulfate, hyaluronate, a collagen (or a hydrolyzed collagen), gelatin, a glycogen, dextran, dextrin, dextran sulfate, cyclodextrin, chitosan, proteoglycan, pullulan, hydroxypropylcellulose, alginic acid, agarose, glycogen, carboxymethyl cellulose, hydroxyethyl cellulose, hydroxypropylcellulose, polyvinylpyrrolidone, polyvinyl alcohol, and a carboxyvinyl polymer. One of these water-soluble macromolecules may be used alone, or a plurality thereof may be used in combination.

### (1-2) Sealing Sheet

The sealing sheet 107 is a sheet for supporting and protecting the conical-projections sheet 105 and is a material having good air permeability, such as, for example, a nonwoven fabric. The sealing sheet 107 is disposed above the substrate 110 of the conical-projections sheet 105 and, furthermore, is fixed therearound to an upper surface of the forming sheet 103. Furthermore, in FIG. 35, a gap is maintained between the conical-projections sheet 105 and the sealing sheet 107. However, as discussed below, both may be fixed together.

A case wherein the sealing sheet 107 includes the base material and the adhesive layer will now be explained. A nonwoven fabric, an elastic cloth, a sponge sheet, or the like can be used as the material of the base material. These materials improve air permeability. In addition, vinyl chloride, a urethane film, an olefin film, or a polyester film can also be used. In addition, air permeability is improved if a plurality of openings is provided in the base material. The adhesive layer includes an acrylic adhesive agent or a silicone-based adhesive agent.

The sealing sheet 107 is fixed to the forming sheet 103 via the adhesive layer. When the conical-projections sheet 105 is stuck to the skin, the sealing sheet 107 and the conical-projections sheet 105, which are fixed to one another via the adhesive layer, are peeled from the forming sheet 103 and, subsequently, the conical-projections sheet 105, together with the sealing sheet 107, is stuck to the skin.

The sealing sheet 107 may be a member including aluminum and a resin layer. In that case, for example, the combinations of layers below are possible.
(1) (Surface side) / resin layer + design layer + aluminum + thermobonding resin layer / (case side)
(2) (Surface side) / resin layer + aluminum + thermobonding resin layer / (case side)
(3) (Surface side) / resin layer + design layer + aluminum + resin layer + thermobonding resin layer / (case side)
(4) (Surface side) / resin layer + aluminum + resin layer + thermobonding resin layer / (case side)
(5) (Surface side) / resin layer + design layer + aluminum + resin layer + aluminum + thermobonding resin layer / (case side)
(6) (Surface side) / aluminum + resin layer + aluminum + thermobonding resin layer / (case side)
(7) (Surface side) / aluminum + thermobonding resin layer / (case side)

Furthermore, if aluminum is used in the sealing sheet 107, then the sealing characteristics of the conical-projections sheet 105 in the packaging body state are improved and, consequently, the microneedles can be protected even if they are made of a material having high hygroscopicity.

A case wherein the sealing sheet includes an aluminum base material and the thermobonding resin layer is explained below. Examples of materials of the thermobonding resin are polyethylene, an ethylene-vinyl acetate copolymer, an ionomer, polypropylene, easily peelable, thermo-adhesive resin (EPR), and the like.

A resin layer may be present between the aluminum base material and the thermobonding resin. In that case, the durability of the aluminum base material is improved.

A design layer may be formed on the aluminum base material. In that case, a design can be provided on the packaging body.

The resin layer may be formed on the aluminum base material or the design layer. In that case, the aluminum base material or the design layer are protected.

The aluminum base material may have a three-layer structure: aluminum + resin + aluminum. In that case, sealing characteristics are improved.

To fix the sealing sheet 107 to the forming sheet 103, the thermobonding resin layer is melted by heating and then is bonded to the forming sheet 103. In that case, the sealing sheet 107 is not fixed to the conical-projections sheet 105.

When the conical-projections sheet 105 is stuck to the skin, first, the sealing sheet 107 is peeled; next, the conical-projections sheet 105 is peeled from the forming sheet 103; last, the conical-projections sheet 105, as a single piece, is stuck to the skin. In addition, subsequently, a lotion pack may be applied on top of the conical-projections sheet 105.

In addition, the explanation of the abovementioned sealing sheet applies likewise to the sealing sheets of the other embodiments.

### (1-3) Forming Sheet

The forming sheet 103 is a member for both forming the microneedle 111 and protecting the microneedle 111 after forming. The forming sheet 103 is stuck to the lower surface of the conical-projections sheet 105. In addition, as shown in FIG. 35, a plurality of micro-recess parts 113, which is for forming and housing the microneedles 111 of the conical-projections sheet 105, is formed on the upper surface of the forming sheet 103. Furthermore, each micro-recess part 113 is an indentation that is open toward the upper side and does not pass through the conical-projections sheet 105.

Taking into consideration that the forming sheet 103 is used as the member that forms the plurality of micro-recess parts 113, the forming sheet 103 consists of, for example, a polyolefin based resin such as polyethylene, polypropylene, and the like. That is, the forming sheet 103 is preferably a hydrophobic sheet wherein the adhesive agent is re-peelable.

### (2-1) Manufacture of Conical-Projections-Sheet, Forming-And-Protecting-Structure Preform

A step of forming the plurality of micro-recess parts 113 on a blank 1107 (a whole forming sheet 103A) is explained below.

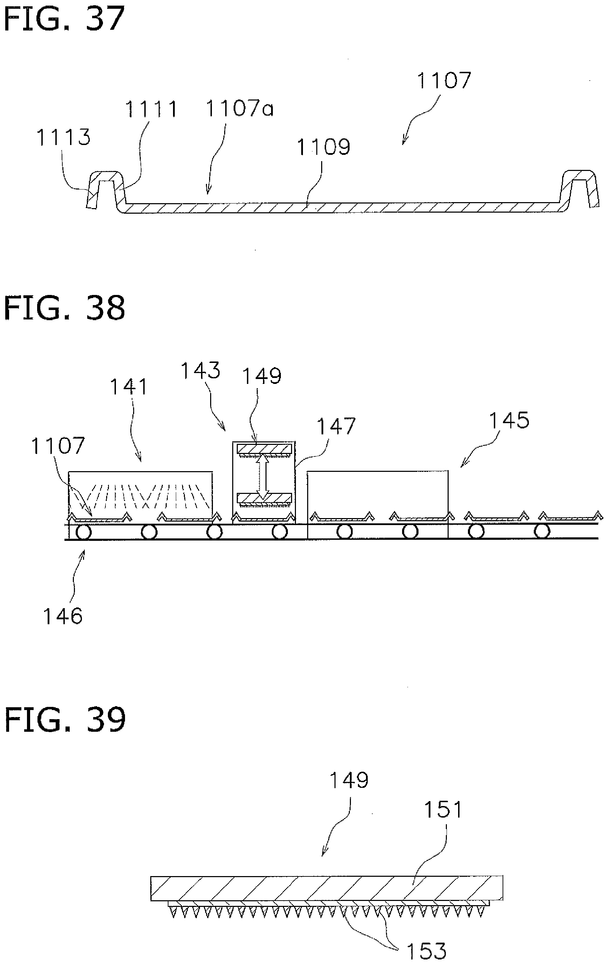

First, as shown in FIG. 36 and FIG. 37, the blank 1107, which will constitute the forming sheet 103, is prepared. FIG. 36 is an oblique view of a blank of a microneedle-forming sheet. FIG. 37 is a cross-sectional view of the blank of the microneedle-forming sheet. As is clear from the figures, the blank 1107 includes a bottom-surface part 1109 and side-surface parts 1111. The bottom-surface part 1109 is, for example, a quadrangular, flat, plate-shaped portion. The side-surface parts 1111 are plate-shaped portions that extend upward from four sides of the bottom-surface part 1109 and form a recessed part 1107a. Furthermore, a folded part 1113 is formed over the entire perimeter at upper ends of the side-surface parts 1111. In a cross section, the folded part 1113 extends toward the outer-perimeter side from the recessed part 1107a and further extends downward.

A process of preheating and forming the blank 1107 will now be explained, with reference to FIG. 38 to FIG. 40. FIG. 38 is a schematic drawing that illustrates the preheating and forming process. As shown in the figure, a heating chamber 141, a forming apparatus 143, and a cooling chamber 145 are provided in series.

In the heating chamber 141, the blank 1107 is preheated prior to being formed. The forming apparatus 143 forms the micro-recess parts 113 on the blank 1107. In the cooling chamber 145, the blank 1107 is cooled. The blank 1107 is transported between the apparatuses by a conveyor 146.

The forming apparatus 143 is an apparatus for forming the plurality of minute micro-recess parts 113 on the bottom-surface part 1109 of the blank 1107. As shown in the figure, the forming apparatus 143 includes a high-precision press 147 and a pincushion-shaped press die 149 (a micro-spike die). As shown in FIG. 39, the press die 149 includes a press part 151. FIG. 39 is a cross-sectional view of the press die. The press part 151 has a shape that matches the bottom-surface part 1109 of the blank 1107, and, on its lower surface, the press part 151 includes a plurality of fabricating projections 153.

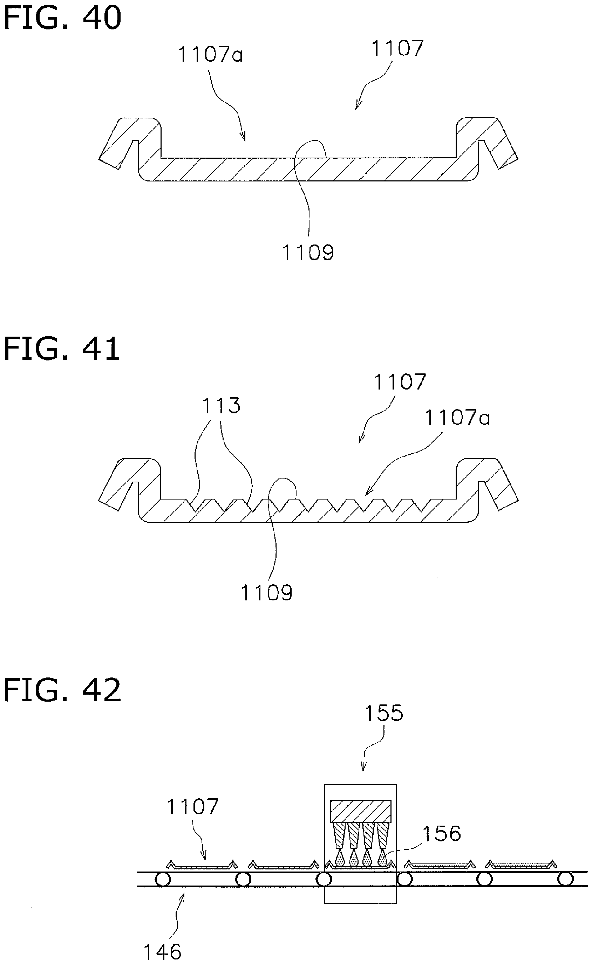

As shown in FIG. 40, prior to the forming work, the bottom-surface part 1109 of the blank 1107 is flat. FIG. 40 is a schematic cross-sectional view that shows a state prior to the forming of the blank. Owing to the preheating prior to the forming, the blank 1107 is already softened when the press die 149 contacts the bottom-surface part 1109. As a result, as shown in FIG. 41, the fabricating projections 153 form the plurality of micro-recess parts 113 on the bottom-surface part 1109. Furthermore, the control of the press is performed by a machine capable of controlling the pressing location, the pressing pressure, the pressing time, and the like with high precision. In addition, although processing time is required, the series of processes may overlap by heating and cooling the press die 149 itself.

FIG. 41 is a schematic cross-sectional view that shows a state after the forming of the blank. As shown in the figure, each micro-recess part 113 has, for example, a conical shape or a pyramidal shape and is open toward the upper side in the figure.

A step of forming a whole conical-projections sheet 105A, which includes the substrate 110 and the plurality of microneedles 111 formed on the lower surface of the substrate 110 and disposed inside the plurality of micro-recess parts 113, by supplying a microneedle material 156 to the blank 1107 (i.e., the whole forming sheet 103A) is explained below.

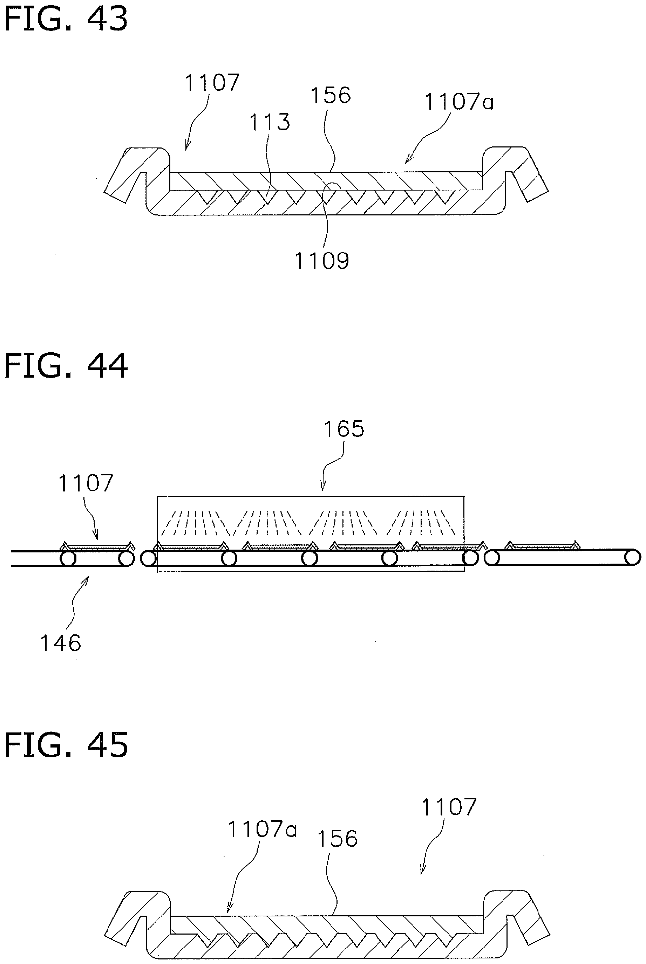

A process of supplying the microneedle material 156 to the blank 1107 will now be explained, with reference to FIG. 42 to FIG. 43. FIG. 42 is a schematic drawing for explaining a process of dropping the microneedle materials onto the microneedle-forming sheets. As shown in the figure, a dispenser 155 is provided. The dispenser 155 is an apparatus that drips the microneedle material 156 onto the recessed part 1107a, thereby filling the recessed part 1107a with the microneedle material 156. The microneedle material 156 is, for example, sodium hyaluronate dissolved in a solvent consisting of water, etc. Furthermore, a medium wherein water-soluble macromolecules in addition to water are soluble is selected as the solvent. Thus, by supplying the microneedle material 156 to the bottom-surface part 1109, the bottom-surface part 1109 is covered by the microneedle material 156, as shown in FIG. 43. FIG. 43 is a schematic cross-sectional view of the microneedle-forming sheet filled with the microneedle material. Furthermore, in the present embodiment, the microneedle material 156 does not penetrate the interiors of the micro-recess parts 113; consequently, there are cases in which air remains in the micro-recess parts 113, in which case a vacuum-evacuating process (not shown) may be supplemented.

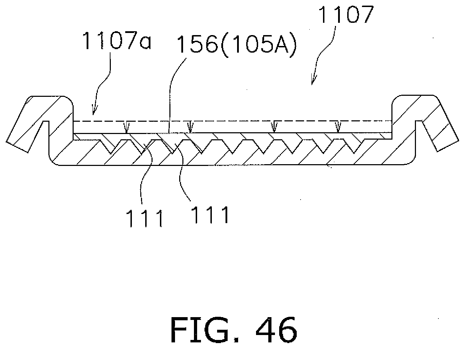

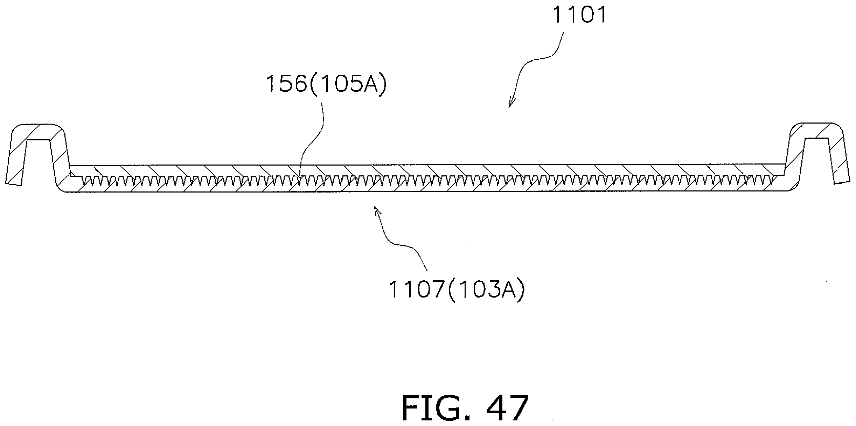

Next, a process of drying and hardening the microneedle material 156 will be explained, with reference to FIG. 44 to FIG. 47. FIG. 44 is a schematic drawing for explaining a process of drying the microneedle materials. As shown in the figure, a drying furnace 165 is provided. In the drying furnace 165, the microneedle material 156 for each of the blanks 1107 is heated and thereby dried. Thereby, the moisture and the solvent agent are evaporated from each microneedle material 156. As a result, starting from the state shown in FIG. 45, the microneedle material 156 shrinks due to the drying, and thereby the volume is reduced to the position shown in FIG. 46. In this state, the microneedle material 156 becomes the whole conical-projections sheet 105A having the plurality of microneedles 111. FIG. 45 is a schematic cross-sectional view of the microneedle-forming sheet filled with the microneedle material prior to the drying process. FIG. 46 is a schematic cross-sectional view of the microneedle-forming sheet filled with the microneedle material after the drying process.

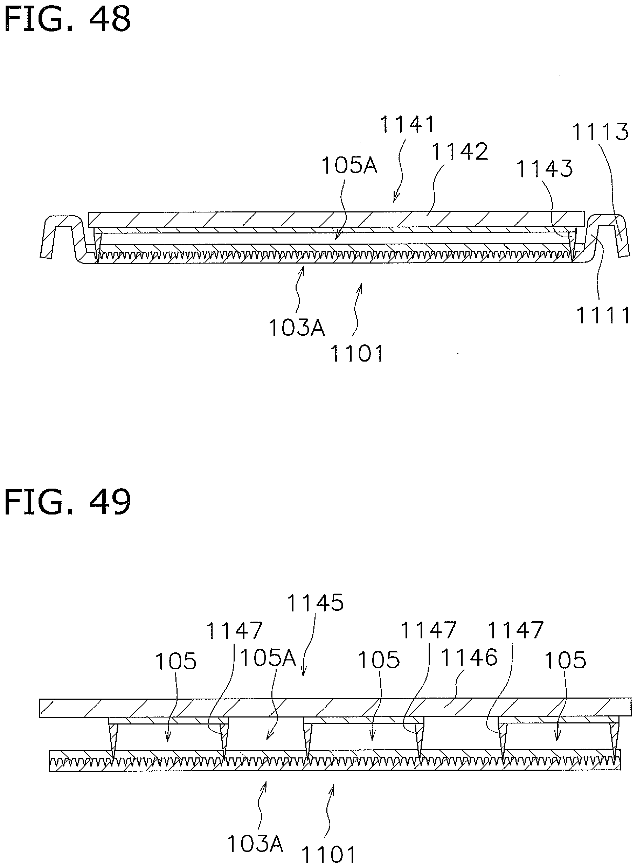

As a result of the above, as shown in FIG. 48, a conical-projections-sheet, forming-and-protecting-structure preform 1101 (hereinbelow, called a preform 1101) is obtained wherein is formed the microneedle material 156 (the whole conical-projections sheet 105A) solidified in the bottom-surface part of the blank 1107 (the whole forming sheet 103A). FIG. 48 is a cross-sectional view of the conical-projections-sheet, forming-and-protecting-structure preform.

### (2-2) Manufacture of Conical-Projections-Sheet Packaging Body

A method of manufacturing the packaging body 101 is explained below, with reference to FIG. 48 to FIG. 55.

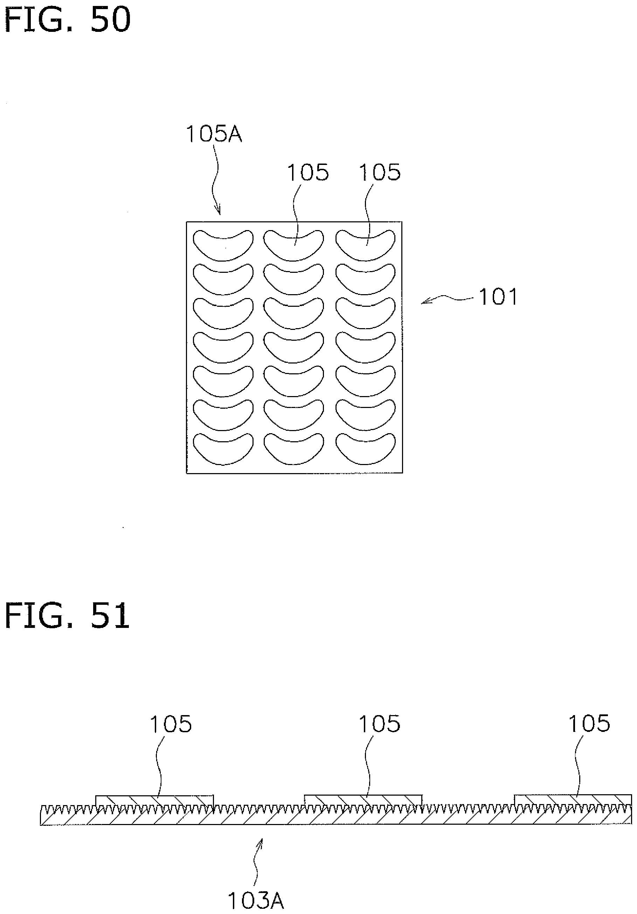

FIG. 48, FIG. 49, FIG. 51, FIG. 53, and FIG. 54 are schematic cross-sectional views for explaining a process of manufacturing the packaging body from the conical-projections-sheet, forming-and-protecting-structure preform. FIG. 50, FIG. 52 and FIG. 55 are plan views of the conical-projections-sheet, forming-and-protecting-structure preform.

First, the preform 1101, wherein the whole conical-projections sheet 105A has been formed on the bottom-surface part of the whole forming sheet 103A, is prepared.

A step of punching out the whole conical-projections sheets 105A such that a desired plurality of the individual conical-projections sheets 105 is formed and then eliminating unnecessary portions is explained below.

As shown in FIG. 48, a cutting apparatus 1141 is used. The cutting apparatus 1141 has a press part 1142 and a ring-shaped cutting blade 1143. The cutting blade 1143 is provided on a lower surface of the press part 1142. The cutting blade 1143 has a shape that corresponds to the outer perimeter of the bottom-surface part of the whole forming sheet 103A. As shown in FIG. 48, when the press part 1142 is lowered and brought proximate to the preform 1101, the cutting blade 1143 cuts the preform 1101 to its lower surface. As a result, the side-surface part 1111 and the folded part 1113 of the whole forming sheet 103A are cut off. As a result, the preform 1101 becomes a planar sheet. Furthermore, a trimming die, such as an etching blade, an engraving blade, or a Thomson blade, can be used as the cutting blade.

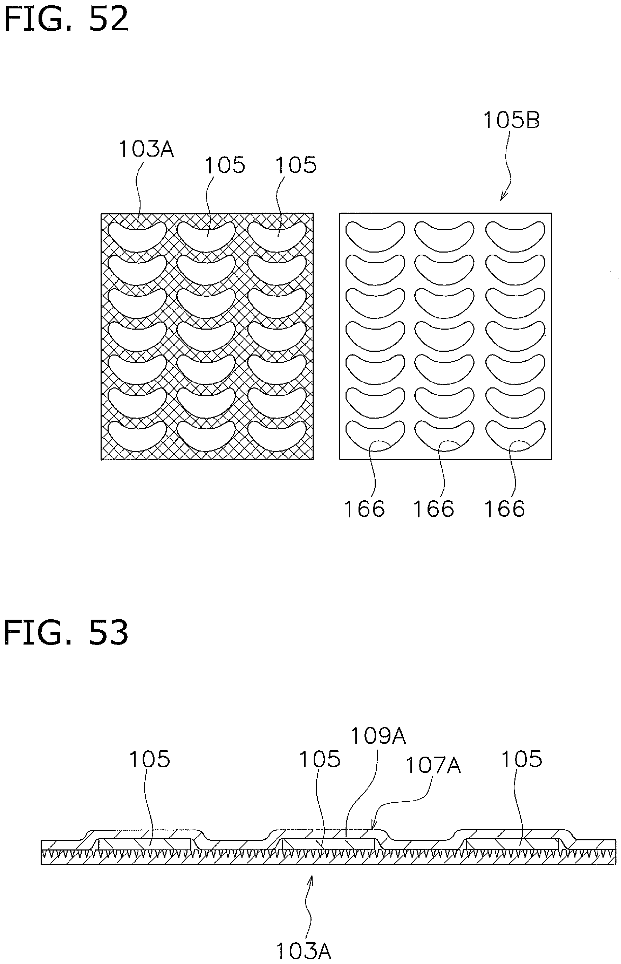

Next, as shown in FIG. 49, a cutting apparatus 1145 is used. The cutting apparatus 1145 includes a press part 1146 and a plurality of cutting blades 1147. Each cutting blade 1147 is provided on a lower surface of the press part 1146. Each cutting blade 1147 is ring shaped. As shown in FIG. 49, when the press part 1146 is lowered and brought proximate to the preform 1101, the cutting blades 1147 cut portions of the solidified whole conical-projections sheet 105A of the preform 1101 to the lower surface, thereby forming the conical-projections sheets 105. However, the cutting blades 1147 do not reach the lower surface of the whole forming sheet 103A; that is, the cutting blades 1147 do not cut the whole forming sheet 103A (performs a half-cut). As a result, as shown in FIG. 51, the plurality of conical-projections sheets 105 punched out in shapes corresponding to the plurality of cutting blades 1147 is obtained on the whole forming sheet 103A. FIG. 51 and the left view in FIG. 52 show a state after waste matter has been removed, and the right view in FIG. 52 shows a waste matter 105B. In the waste matter 105B, trim remnants 66, which are the remnants remaining after the plurality of conical-projections sheets 105 has been eliminated, are formed.

Next, a step of fixing a whole sealing sheet 107A to the plurality of individual conical-projections sheets 105 will be explained.

As shown in FIG. 53, the whole sealing sheet 107A, whose plane is a quadrangle corresponding to the whole forming sheet 103A, is fixed to each of the conical-projections sheets 105. That is, the whole sealing sheet 107A is stuck across the entire surface of the whole forming sheet 103A. Furthermore, the whole sealing sheet 107A is fixed to the whole forming sheet 3A at the periphery of each individual conical-projections sheet 105.

At this time, the whole sealing sheet 107A is stuck not only to the conical-projections sheets 105 but also to exposed parts of the upper surface of the whole forming sheet 103A. That is, in the step of fixing the whole sealing sheet 107A, the whole sealing sheet 107A is fixed to the whole forming sheet 3A at the periphery of each individual conical-projections sheet 105.

Last, a step will be explained wherein, in units of the individual conical-projections sheet 105, the whole forming sheet 3A and the whole sealing sheet 107A are punched out, thereby forming a plurality of the conical-projections-sheet packaging bodies 101, each packaging body 101 including an individual conical-projections sheet 105, an individual forming sheet 103, and an individual sealing sheet 107.

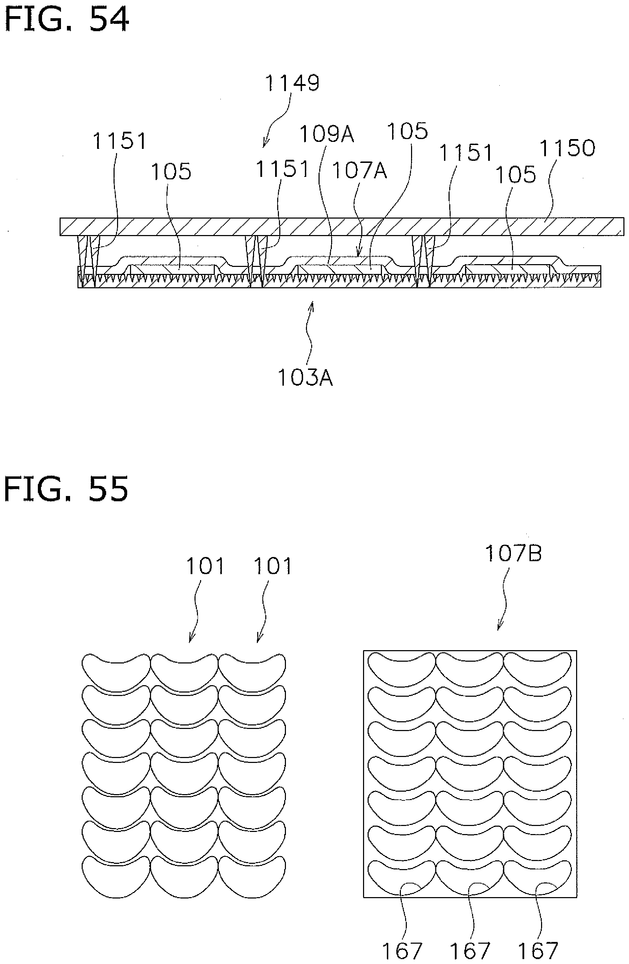

As shown in FIG. 54, a cutting apparatus 1149 is used. The cutting apparatus 1149 includes a press part 1150 and a plurality of cutting blades 1151. The cutting blades 1151 are provided on a lower surface of the press part 150. Each cutting blade 1151 is ring shaped and has a diameter larger than that of the cutting blade 1147. When the press part 1150 is lowered and brought proximate to the whole forming sheet 103A and the whole sealing sheet 107A, the cutting blades 1151 cut, to the lower surface, the whole forming sheet 103A and the whole sealing sheet 107A at the outer perimeters of the individual conical-projections sheets 105. As a result, as shown on the left side in FIG. 55, a plurality of the packaging bodies 101 is obtained. Furthermore, the drawing on the right side of FIG. 55 is waste matter 107B. In the waste matter 107B, trim remnants 167, which are the remnants remaining after the plurality of packaging bodies 101 has been cut out, are formed.

In the packaging body 101 manufactured by this manufacturing method, the conical-projections sheets 105 are formed in the forming sheet 103 and, furthermore, their state at the time when they are formed is maintained. Specifically, the plurality of microneedles 111 of each conical-projections sheet 105 is formed by the plurality of micro-recess parts 113 of the forming sheet 103; subsequently, too, the plurality of microneedles 111 is protected by the plurality of micro-recess parts 113. That is, the plurality of microneedles 111 of each conical-projections sheet 105 is reliably protected.

In addition, because the packaging body 101 includes the sealing sheet 107, which corresponds to the conical-projections sheet 105 and is fixed to the forming sheet 103, each individual conical-projections sheet 105 is protected.

Furthermore, in the present manufacturing method, the plurality of conical-projections-sheet packaging bodies 101 is formed all at once by the last article-punching step (FIG. 54). Accordingly, mass production with a small number of processes becomes possible.

Furthermore, in the present manufacturing method, the whole sealing sheet 107A is fixed to the whole forming sheet 103A at the periphery of each conical-projections sheet 105, and therefore the last article-punching step can create the state wherein the individual sealing sheets 107 are fixed to the individual forming sheets 103 at the peripheries of the individual conical-projections sheets 105. Thereby, the individual conical-projections sheets 105 are protected from outside contamination.

As a result of the above, in the state wherein the conical-projections-sheet packaging bodies 101 are completed, the conical-projections sheets 105 are fixed to the forming sheets 103 by the sealing sheets 107. Thus, the microneedles 111 are protected when the conical-projections-sheet packaging bodies 101 are transported or used.

As discussed above, the forming sheet 103 functions as a forming die for forming the microneedles 111 of each conical-projections sheet 105. In addition, the forming sheet 103 functions as a protective material that protects the microneedles 111 of each conical-projections sheet 105.

### INDUSTRIAL APPLICABILITY

The present invention can be broadly applied to a conical-projections-sheet packaging body including a plurality of conical projections and to a method of manufacturing the same.

### REFERENCE SIGNS LIST

- 1: Conical-projections-sheet packaging body
- 3: Support-body-attached, conical-projections sheet
- 5: Packaging material
- 7: Conical-projections forming sheet
- 9: Sealing sheet
- 21: Support body
- 23: Conical-projections sheet
- 23a: Substrate
- 23b: Conical projection
- 23c: Surface
- 31: Housing part
- 31a: Recessed-housing part
- 33: Bottom surface
- 33a: Conical-recess part
- 35: Stepped surface
- 37: Flat surface

## Claims

1. A conical-projections-sheet packaging body, comprising:
a conical-projections sheet including a substrate and a plurality of conical projections formed on a surface of the substrate;
a conical-projections forming sheet that has a plurality of conical-recess parts, the plurality of conical-recess parts being used to form the plurality of conical projections and where the plurality of conical projections is disposed, and that protects the plurality of conical projections in a state wherein the plurality of conical projections is tightly adhered to the surface of the substrate; and
a sealing sheet that, by being fixed to the conical-projections forming sheet, seals the conical-projections sheet between the conical-projections forming sheet and the sealing sheet.

2. The conical-projections-sheet packaging body according to claim 1, wherein
the conical-projections forming sheet has a recessed-housing part wherein the plurality of conical-recess parts is formed; and
the conical-projections sheet is disposed inside the recessed-housing part.

3. The conical-projections-sheet packaging body according to claim 1 or claim 2, further comprising:
a support body that, by being fixed to a second surface of the substrate of the conical-projections sheet and further being adhered to the conical-projections forming sheet, supports a state wherein the plurality of conical projections of the conical-projections sheet is disposed inside the plurality of conical-recess parts of the conical-projections forming sheet.

4. The conical-projections-sheet packaging body according to claim 3, wherein
a process of reducing peel strength is performed on a portion of the conical-projections forming sheet at which the support body is adhered.

5. The conical-projections-sheet packaging body according to claim 4, wherein
the process of reducing the peel strength is performed by applying a die-releasing agent or using a material that exhibits die-releasing effect, or both.

6. The conical-projections-sheet packaging body according to claim 4 or claim 5, wherein
the peel strength between the support body and the sealing sheet is 2.0-6.5 (N/10 mm).

7. The conical-projections-sheet packaging body according to any one of claim 4 through claim 6, wherein
the peel strength between the support body and the conical-projections forming sheet is 0.5-4.0 (N/10 mm).

8. A method of manufacturing a conical-projections-sheet packaging body, comprising:
forming a plurality of conical-recess parts on a conical-projections forming sheet;
forming, by supplying a conical-projections material to the conical-projections forming sheet, a conical-projections sheet including a substrate and a plurality of conical projections formed on a surface of the substrate and disposed inside the plurality of conical-recess parts; and
sealing the conical-projections sheet between the conical-projections forming sheet and a sealing sheet by fixing the sealing sheet to the conical-projections forming sheet.

9. The method of manufacturing the conical-projections-sheet packaging body according to claim 8, further comprising:
forming, prior to forming the plurality of conical-recess parts, a recessed-housing part at a location at which the plurality of conical-recess parts is to be formed on the conical-projections forming sheet;
wherein,
in the step of forming the conical-projections sheet, the conical-projections sheet is formed inside the recessed-housing part.

10. The method of manufacturing the conical-projections-sheet packaging body according to claim 8 or claim 9, further comprising:
maintaining, by fixing a support body to a second surface of the substrate of the conical-projections sheet and further adhering the support body to the conical-projections forming sheet, a state wherein the plurality of conical projections of the conical-projections sheet is disposed inside the plurality of conical-recess parts of the conical-projections forming sheet.

11. The method of manufacturing the conical-projections-sheet packaging body according to claim 10, further comprising:
performing, prior to the step of forming the conical-projections sheet, a process of reducing peel strength at a portion of the conical-projections forming sheet at which the support body is to be adhered.

12. The method of manufacturing the conical-projections-sheet packaging body according to claim 11, wherein
the process of reducing the peel strength is performed by applying a die-releasing agent or using a material that exhibits a die-releasing effect, or both.
